# EUROPEAN PATENT APPLICATION

(11) **EP 4 293 109 A1**
(43) Date of publication of application: **20.12.2023**
(21) Application number: 22179041.3
(22) Date of filing: 14.06.2022
(51) Int. Cl.: C12N 5/077, A23L 13/00

(54) **NON-SKELETAL MUSCLE-DERIVED CELLS AS A SOURCE OF SUSPENSION CAPABLE MYOGENIC CELLS FOR CULTURED FOODS**

(71) Applicant: Upside Foods, Inc., Berkeley CA 94710 (US)
(72) Inventor: The designation of the inventor has not yet been filed
(74) Representative: Mewburn Ellis LLP

(57) **Abstract**

Provided herein are methods relating to making non-natively occurring myogenic cells. For example, provided herein are methods for transdifferentiating a liver-derived cell to a non-natively occurring myogenic cell. In another example, the method relates to using dedifferentiation to make non-natively occurring myogenic cells.

## Description

### 1. BACKGROUND OF THE INVENTION

Livestock production systems as we know them today are the result of an interaction between domestic animals and the environment, modulated by human activities, that date back to Neolithic times. Animal agriculture uses more than three-quarters of the world's agricultural land (Foley et al. 2011; Poore and Nemecek 2018). As a result, the impact of agriculture, forestry and related land use is extensive and far reaching. It accounts for 18.4% of global greenhouse gas emissions and animal farming quite possibly may be the largest human-related source of water pollution.

The challenges from climate change and the consequences on food security and the agriculture socio-economy will vary across the globe, however the consensus is that it will have profound impact on world hunger and access to animal protein. Rising temperatures, drought and increase in frequency of extreme weather events will greatly affect available land for agriculture, irrigation, and supply chains to further drive the cost of already expensive sources of animal protein even higher.

Livestock farming has an immense carbon footprint and deleterious effects on climate change from deforestation to transport, waste management to food storage, each step of the food chain brings with it a higher carbon footprint. Feeding the world's billions of people every day is a massive task and one that is likely to get even greater as human populations rise and we increasingly feel the effects of climate change.

If the world is to meet the ambition of reaching net zero carbon emissions by the middle of the century as outlined in the Paris Agreement on climate change, the food industry will have to play its part. All this and more clearly indicates that new ways to provide protein sources without needing to rear, slaughter and butcher livestock, which not only reduce the impact of livestock farming on our environment but are also insulated from the effects of climate change, are highly important.

### 2. SUMMARY OF THE INVENTION

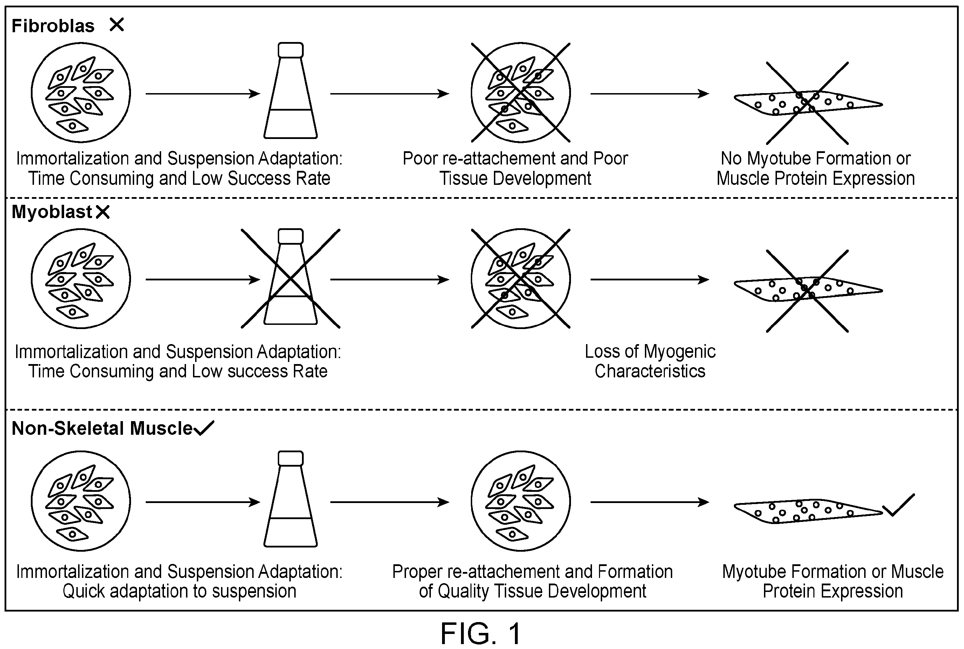

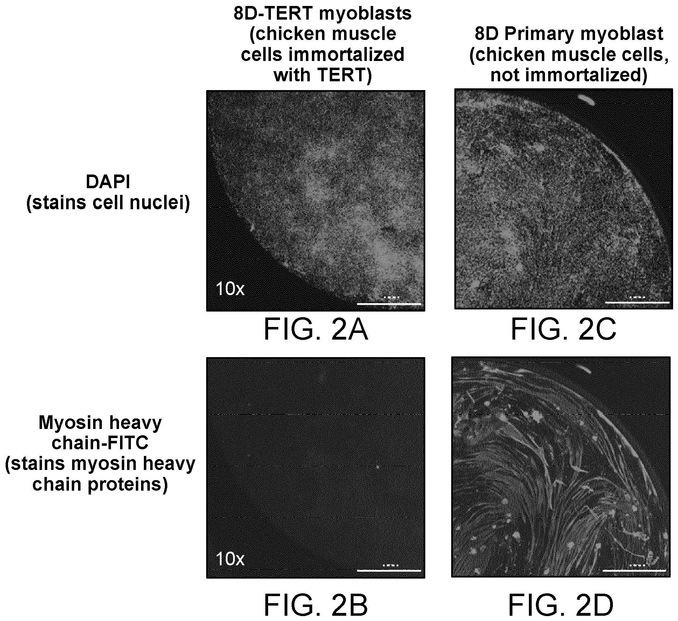

This disclosure features methods for transdifferentiating non-muscle liver-derived cells to non-natively occurring myogenic cells suitable for consumption. These methods provide a source of cells capable of undergoing spontaneous immortalization and/or adaptation to suspension culture without compromising the cell's ability to form myogenic cells, which is unlike other sources of cells previously used to make myogenic cells suitable for consumption (see, e.g., FIG. 1 comparing attempts using fibroblasts and myoblasts with non-muscle liver-derived cells). As noted to in FIG. 1, the distinctions between the sources of starting cells are important because traditional cell line adaptation into desired media (e.g., low-cost media) and culture format (i.e., suspension culture) takes long periods (e.g., 1-3 months) and typically requires immortalization. Unfortunately, immortalization of muscle cells using Telomerase Reverse Transcriptase (TERT) often results in loss of differentiation capacity (e.g., decreases in MyHC expression and decreased ability to form myotubes) (see, **FIGs. 2A-2D****),** thereby limiting the usability of these cells as a source for differentiating into non-naturally occurring myogenic cells suitable for consumption. In contrast, the methods provided herein not only provide a source of cells free of the problems associated with using fibroblasts and myoblasts as the source of cells, but also produce myogenic cells with higher total protein as compared to controls, including chicken fibroblasts grown in adherent conditions. Overall, this work demonstrates that transdifferentiation of non-muscle, liver-derived cells can serve as a robust method of making non-natively occurring myogenic cells that are suitable for consumption.

### 3. BRIEF DESCRIPTION OF THE DRAWINGS

These and other features, aspects, and advantages of the present invention will become better understood with regard to the following description, and accompanying drawings, where:
**FIG. 1** provides a schematic of experimental plans for comparing efficacy of myotube formation and muscle protein expression from fibroblasts, myoblasts, and non-skeletal muscle cells (e.g., liver-derived cells). Top panel shows the characteristics associated with using fibroblast to make myogenic cells. Middle panel shows the characteristics associated with using myoblasts to make myogenic cells. Bottom panel shows the characteristics associated with the present disclosure showing that non-muscle cells (e.g., liver derived cells) provide a robust alternative to making myogenic cells suitable for consumption as compared to methods starting with fibroblasts (Top panel) or other muscle cells (Middle panel).
**FIGs. 2A-2D** shows representative images of myotube formation in chicken myoblasts. **FIGs. 2A-2B** show myotube formation in chicken myoblasts immortalized with TERT (8D-TERT): DAPI staining in **FIG. 2A** and myosin heavy chain-FITC staining in **FIG. 2B. FIGs. 2C-2D** show myotube formation in chicken myoblasts not immortalized with TERT (8D primary myoblasts): DAPI staining in **FIG. 2A** and myosin heavy chain-FITC staining in **FIG. 2B****.**

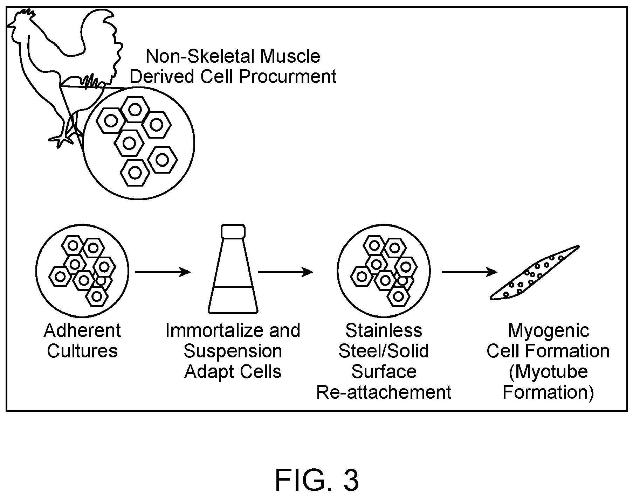

**FIG. 3** provides a schematic of an exemplary embodiment where non-skeletal muscle derived cells (e.g., liver-derived cells) are procured from the species of interest (e.g., chicken) and selected in adherent culture. Cells are then immortalized and adapted for suspension culture. The immortalized suspension cells are screened for reattachment to solid surface (plastic/stainless steel) and assayed for myotube formation and expression of meat proteins.

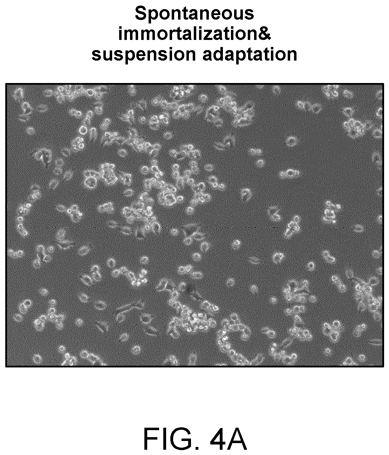

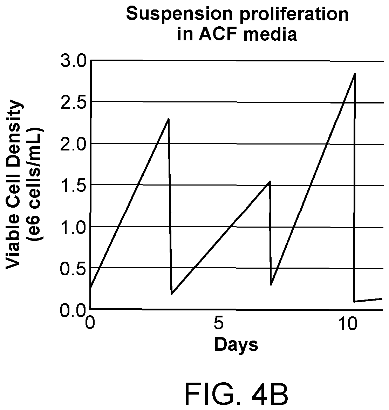

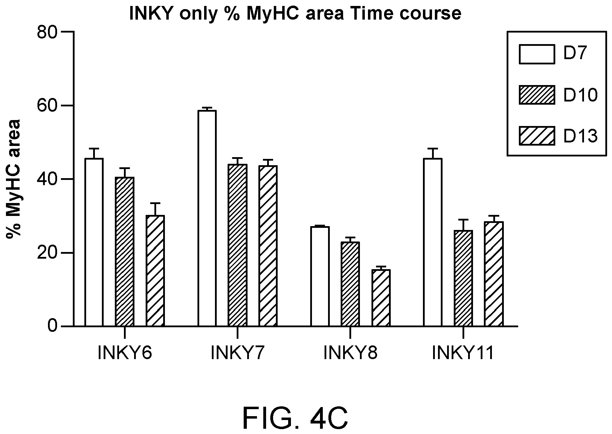

**FIGs. 4A-4C** show proof of concept data from rodentia liver-derived Inky/Kawhi cell lines cultured according to the methods described herein. **FIG. 4A** shows a representative image of rodentia liver-derived cell lines (Inky/Kawhi) in suspension showing adaptation to suspension culture. **FIG. 4B** shows a representative viable cell count for a liver-derived cell line (Inky) over a 10-day period of suspension culture. **FIG. 4C** shows quantification of MyHC staining represented as % MyHC area for each of four liver-derived cell lines (Inky only) at three different time points.

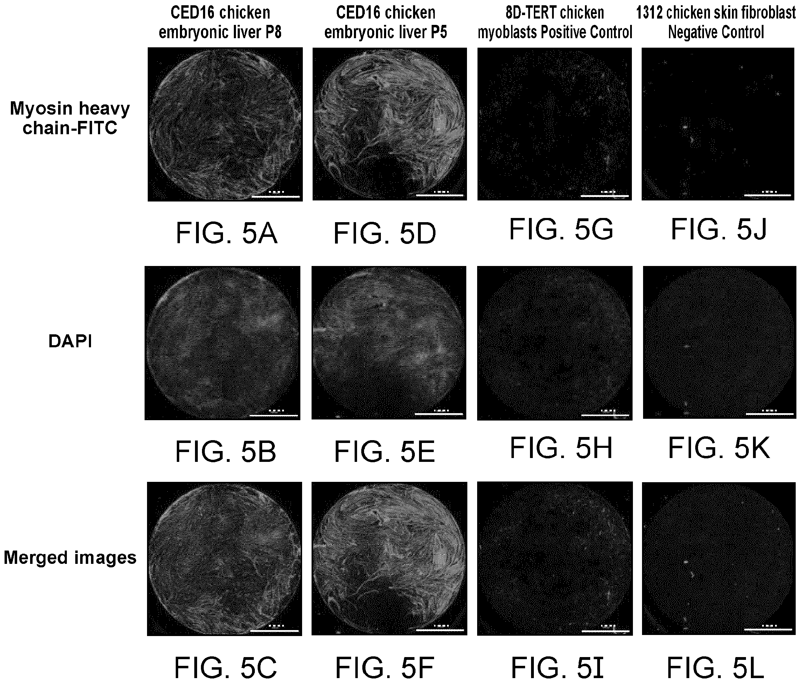

**FIG. 5A-5L** shows representative images of MyHC staining and myotube formation in chicken embryonic liver-derived cells P5 - passage 5 **(****FIGs. 5A-C****)** or P8 - passage 8 **(****FIGs. 5D-F****),** positive control chicken myoblasts immortalized with TERT (e.g., 8D-TERT cells) **(****FIGs. 5G-5I****),** negative control chicken skin fibroblasts (e.g., 1312 cells) **(****FIGs. 5J-5L****)** following three days in proliferation media and 4 days in differentiation media. **FIGs. 5A, 5D, 5G,** and **5J** show MyHC staining with myotubes indicated as elongated tendrils. **FIGs. 5B, 5E, 5H,** and **5K** show DAPI (blue signal) for nuclei. **FIGs. 5C, 5F, 51,** and **5L** show merged images of MyHC stain and DAPI stain. Abbreviations: P - indicates passage number; CED16 - chicken embryonic day 16 liver cells; 8D-TERT - chicken myoblasts immortalized with TERT; and 1312 - chicken skin fibroblasts immortalized with TERT.

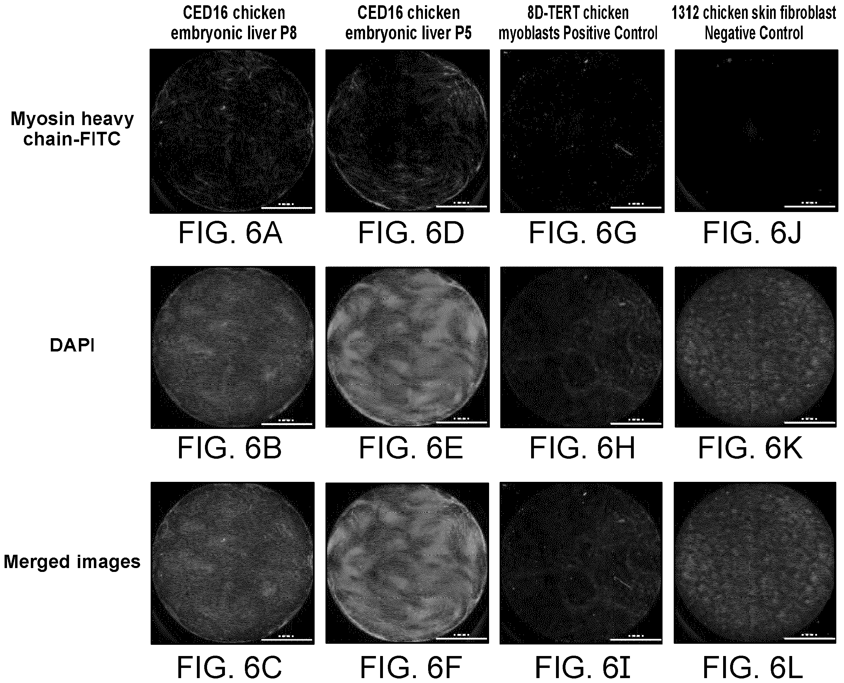

**FIG. 6A-6L** shows representative images of MyHC staining and myotube formation in chicken embryonic liver-derived cells P5 - passage 5 **(****FIGs. 6A-C****)** or P8 - passage 8 **(****FIGs. 6D-F****),** positive control chicken myoblasts immortalized with TERT (e.g., 8D-TERT cells) **(****FIGs. 6G-6I****),** negative control chicken skin fibroblasts (e.g., 1312 cells) **(****FIGs. 6J-6L****)** following three days in proliferation media and 4 days in differentiation media. **FIGs. 6A, 6D, 6G,** and **61** show MyHC staining with myotubes indicated as elongated tendrils. **FIGs. 6B, 6E, 6H,** and **6J** show DAPI (blue signal) for nuclei. **FIGs. 6C, 6F, 61,** and **6K** show merged images of MyHC stain and DAPI stain. Abbreviations: P - indicates passage number; CED16 - chicken embryonic day 16 liver cells; 8D-TERT - chicken myoblasts immortalized with TERT; and 1312 - chicken skin fibroblasts immortalized with TERT.

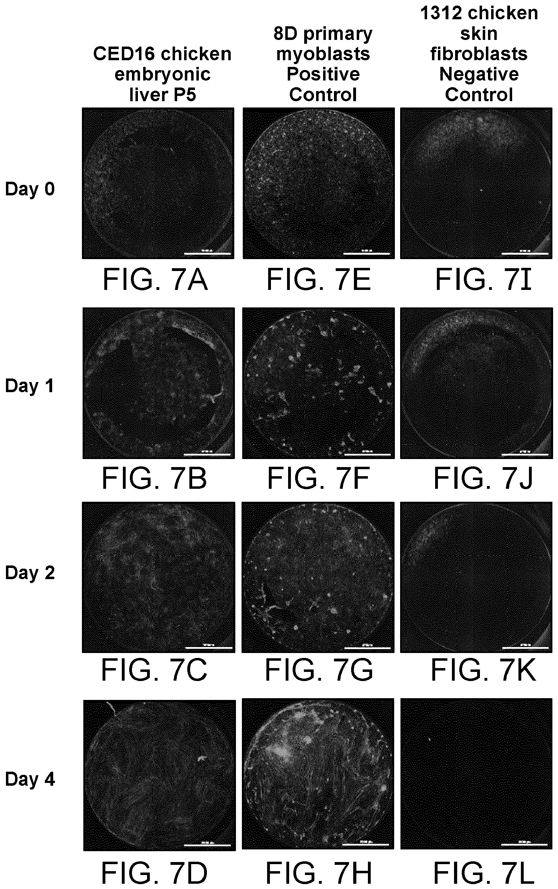

**FIG. 7A-7L** shows representative images of a time course of MyHC staining and myotube formation in chicken embryonic liver-derived cells P5 - passage 5 **(****FIGs. 7A-****D),** positive control 8D primary myoblasts **(****FIGs. 7E-7H****),** negative control chicken skin fibroblasts (e.g., 1312 cells) **(****FIGs. 7I-7L****).** Following three days in proliferation media each condition was analyzed for MyHC expression and DAPI. Merged images show a combination of MyHC and DAPI stains for each condition. **FIGs. 7A, 7E,** and **71** show images of MyHC/DAPI staining at day 3 (same day as switch to differentiation media) with myotubes indicated as elongated tendrils. **FIGs. 7B, 7F,** and **7J** show images of MyHC/DAPI staining at day 4 (day 1 following switch to differentiation media) with myotubes indicated as elongated tendrils. **FIGs. 7C, 7G,** and **7K** show images of MyHC/DAPI staining at day 5 (day 2 following switch to differentiation media) with myotubes indicated as elongated tendrils. **FIGs. 7D, 7H,** and **7L** show images of MyHC/DAPI staining at day 7 (day 4 following switch to differentiation media) with myotubes indicated as elongated tendrils.

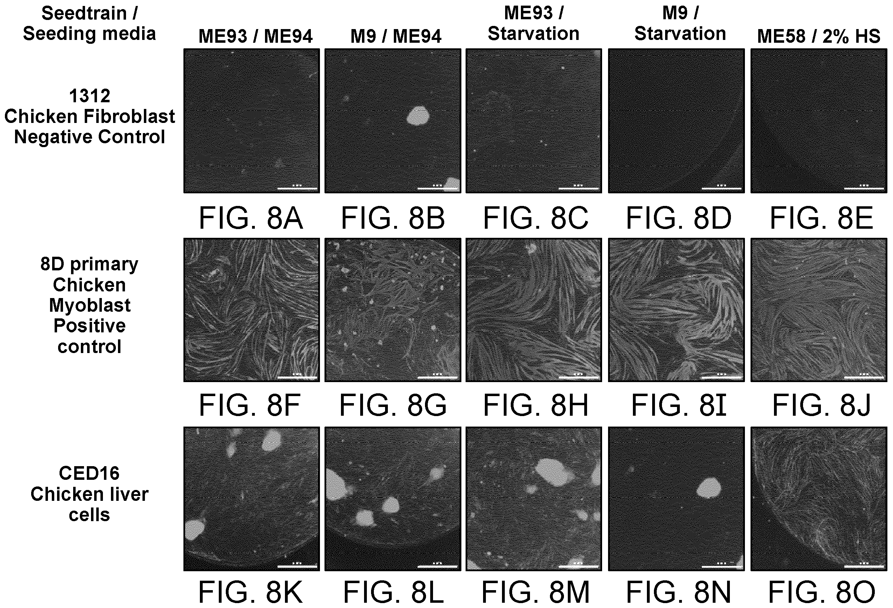

**FIG. 8A-O** shows representative images of MyHC staining and myotube formation for negative control chicken skin fibroblasts (e.g., 1312 cells) **(****FIGs. 8A-8E****),** positive control 8D primary myoblasts **(****FIGs. 8F-8J****),** and chicken embryonic liver-derived cells P5 - passage 5 **(****FIGs. 8K-8O****)** cultured in various combinations of seedtrain and seeding media. **FIGs. 8A, 8F,** and **8K** show images of MyHC/DAPI staining at day 7 for ME 93 / ME94. **FIGs. 8B, 8G,** and **8L** show images of MyHC/DAPI staining at day 7 for ME 9 / ME94. **FIGs. 8C, 8H,** and **8M** show images of MyHC/DAPI staining at day 7 for ME 93 / starvation. **FIGs. 8D, 81,** and **8N** show images of MyHC/DAPI staining at day 7 for ME 9 / starvation. **FIGs. 8E, 8J,** and **8O** show images of MyHC/DAPI staining at day 7 for ME 58 / 2% horse serum. Myotubes are indicated as elongated tendrils.

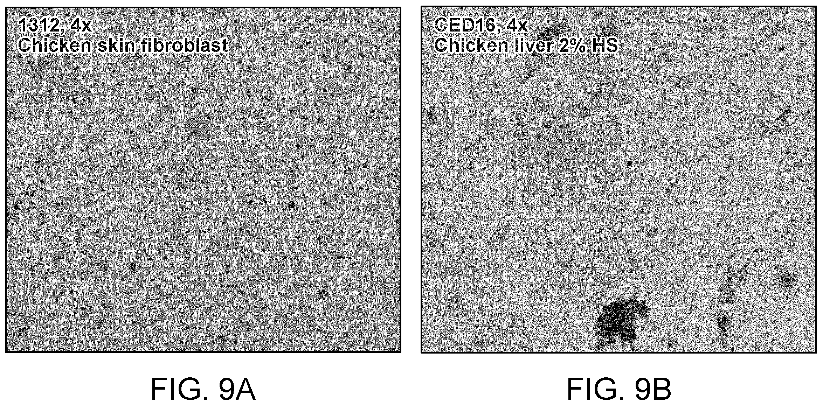

**FIGs. 9A-9B** shows representative bright field images of chicken skin fibroblasts (e.g., 1312 cells) **(****FIG. 9A****)** and chicken embryonic liver-derived cells **(****FIG. 9B****)** cultured in roller bottles.

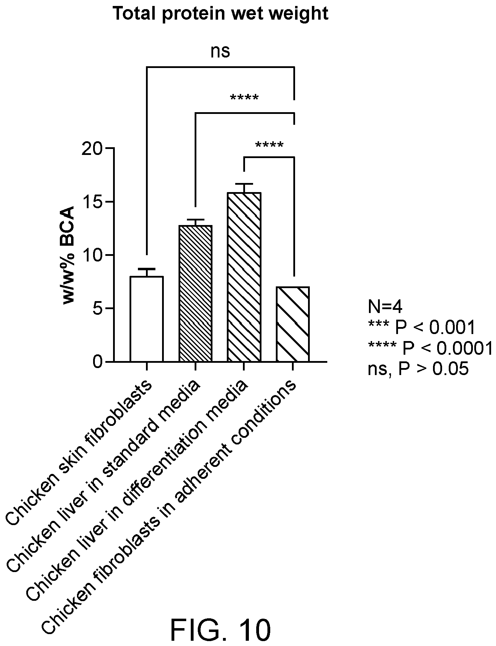

**FIG. 10** is a bar graph showing total protein wet weight (w/w% BCA) for chicken skin fibroblasts (e.g., 1312 cells), non-natively occurring myogenic cells transdifferentiated from chicken embryonic liver cells that were cultured in standard media ("Chicken liver in standard media") or differentiation media ("Chicken liver in differentiation media"), and chicken fibroblasts grown in adherent conditions.

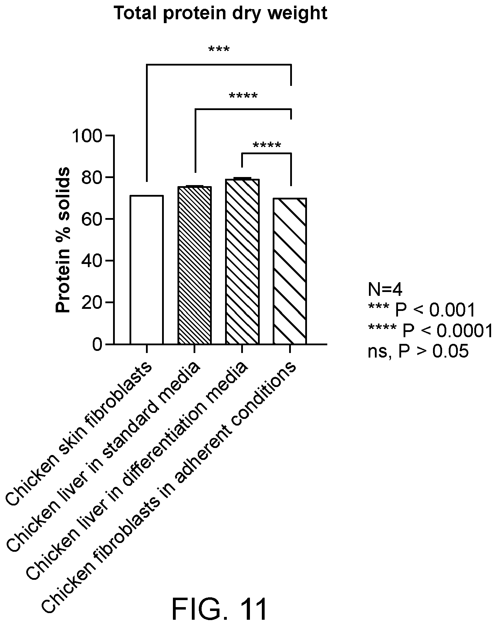

**FIG. 11** is a bar graph showing total protein dry weight (Protein % solids) for chicken skin fibroblasts (e.g., 1312 cells), non-natively occurring myogenic cells transdifferentiated from chicken embryonic liver cells that were cultured in standard media ("Chicken liver in standard media") or differentiation media ("Chicken liver in differentiation media"), and chicken fibroblasts grown in adherent conditions.

### 4. DETAILED DESCRIPTION OF THE INVENTION

This disclosure features methods for transdifferentiating a non-muscle liver-derived cell to a non-natively occurring myogenic cell suitable for consumption without compromising necessary differentiation capacity. In particular, this disclosure is based in part on the discovery that transdifferentiating non-muscle liver-derived cells to a non-natively occurring myogenic cell produces myogenic cells having statistically significantly greater total protein content than compared to chicken fibroblasts grown in adherent conditions.. A non-limiting example of the methods provided herein are shown in FIG. 3. Non-muscle liver-derived cells are procured from the species of interest (e.g., chicken) and selected in adherent culture. Cells are then immortalized (e.g., through spontaneous immortalization) and adapted for suspension culture. The immortalized suspension cells are screened for reattachment to solid surface (plastic/stainless steel) and assayed for myotube formation and expression of meat proteins (e.g., Myosin heavy chain (MyHC)). During these step, the non-muscle liver-derived cells transdifferentiate to non-natively occurring myogenic cells suitable for consumption.

### 4.1. Definitions

Unless defined otherwise, all technical and scientific terms used herein have the same meaning as is commonly understood by one of ordinary skill in the art to which this disclosure belongs. In the event that there is a plurality of definitions for terms cited herein, those in this section prevail unless otherwise stated.

Throughout this disclosure, the term "a" or "an" entity refers to one or more of that entity. As such, the terms "a" (or "an"), "one or more," and "at least one" can be used interchangeably herein. Furthermore, "and/or" as used in a phase such as "A and/or B" herein is intended to include "A and B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

As used herein, the terms "comprising" and "including" or grammatical variants thereof are to be taken as specifying the stated features, integers, steps, or components but do not preclude the addition of one or more additional features, integers, steps, components, or groups thereof. This term encompasses the terms "consisting of' and "consisting essentially of'.

As used herein, the terms "cell" and "cell line" are sometimes used interchangeably. As used herein, the term "cell" can refer to one or more cells originating from a cell line. As used herein, the term "cell line" can refer to a population of cells.

As used herein, the term "cultivation infrastructure" refers to the environment in which liver-derived cells, dedifferentiated cell, or myogenic cells (e.g., non-natively occurring myogenic cells) are cultured (i.e., the environment in which the myogenic cell is cultivated).

As used herein, the term "dedifferentiated," "dedifferentiation," "dedifferentiating," and "loss of differentiation" refers to the conversion of a cell into a cell having greater differentiation potential. For example, a somatic cell (i.e., a differentiated liver cell) is converted to a cell having that has the capacity to self-renew by dividing and to develop into multiple cell types present in the liver or even cells of a separate lineage (e.g., myogenic cells).

As used herein, the term "differentiation capacity" refers to a cell's ability to differentiate to a particular cell lineage, stem cell, progenitor cell, or terminally differentiated cell. As used herein, the term "myogenic differentiation capacity" refers to a cell's ability to differentiate to a myogenic cell.

As used herein, the terms "hepatic stem cell" or "liver stem cell" refer to cells responsible for normal tissue turnover, cells which give rise to regeneration after partial hepatectomy, cells responsible for progenitor-dependent regeneration, cells which produce hepatocyte and bile duct epithelial phenotypes in vitro, and transplantable liver repopulating cells. For example, the existence of stem cells within adult hepatic tissue is reviewed in Navarro-Alvarez et al., MethodsMol Biol. 2010; 640:181-236.

As used herein, the term "immortalized cell" refers to cells that are passaged or modified to proliferate indefinitely and evade normal cellular senescence.

As used herein, the terms "liver-derived cell" refers to a cell originating from the liver, liver tissue, the hepatic vascular system, the biliary system (e.g., bile duct), and the architecture of hepatic tissue (e.g., connective tissue). A "liver-derived cell" can be a cell that while originating from the liver, liver tissue, the hepatic vascular system, the biliary system (e.g., bile duct), and/or the architecture of hepatic tissue (e.g., connective tissue) is no longer a phenotypic liver cell (e.g., no longer express markers associated with a liver cell).

As used herein, the term "multipotent stem cells" when used in reference to the liver refers to liver cells that have the capacity to self-renew by dividing and to develop into multiple specialized cell types present in the liver.

As used herein, the term "myoblast" refers to mononucleated muscle cells. They are embryonic precursors of myocytes, also called muscle cells. Although myoblasts may be classified as skeletal muscle myoblasts, smooth muscle myoblasts, and cardiac muscle myoblasts depending on the type of muscle cell that they will differentiate into, in this specification the term myoblasts refer to skeletal muscle myoblasts.

As used herein, the term "myofibroblast" when used in reference to the liver refers to cells including exocytotic vesicles, stress fibers, and/or smooth muscle actin staining.

As used herein, the term "myotube" refers to elongated structures, the result of differentiated myoblast. Upon differentiation, myoblasts fuse into one or more nucleated myotubes and express skeletal muscle markers.

As used herein, the terms "non-naturally occurring," "non-natively occurring," and "unorthodox" when used in reference to a myogenic cell of the present disclosure refers to a non-native myogenic cell. For example, a "non-natively occurring myogenic cell" refers to myogenic cell types used for cultured food production, including myocytes, myoblasts, myotubes, multinucleated myotubes, satellite cells, skeletal muscle fibers, or any combination thereof that are not natively present in an animal. As described herein, a liver-derived cell is transdifferentiated to a cell that is myogenic and non-native to the animal because in the animal it existed as a liver-derived cell (e.g., a liver derived cell as described herein.

As used herein the term "passaged cell" refers to the number of times the cells in the culture have been sub-cultured. This may occur without consideration of the inoculation densities or recoveries involved.

As used herein, the term "population doubling level (PDL)" refers to the total number of times the cells in the population have doubled since their primary isolation *in vitro.* Mathematically this is described as n = 3.32 (log UCY - log 1) + X, where n = the final PDL number at end of a given subculture, UCY = the cell yield at that point, 1 = the cell number used as inoculum to begin that subculture, and X = the doubling level of the inoculum used to initiate the subculture being quantitated. Here, primary isolation *in vitro* refers to obtaining the population of liver-derived cells from the animal.

As used herein, the term "skeletal muscle progenitor cell" (SMPC), also referred to herein as "myogenic progenitors," can contribute to muscle regeneration, differentiate into skeletal muscles, and are valuable cells for therapeutic application.

As used herein, the term "substantially free of' or "substantially free from" means the amount (e.g., absolute number within a population or concentration/percentage within a population) of a cell or cell type is below a value where the cell or cell type, or any cell derived therefrom, could contribute to the population. For example, a population substantially free of a cell means that upon differentiation of the population the cell does not contribute progeny to the differentiated population.

As used herein, the terms "transdifferentiation," "transdifferentiating" and "re-differentiating" refers to the conversion of a cell type present in one tissue or organ into a cell type from another tissue or organ without going through a pluripotent cell state. In other cases, transdifferentiation can be induced using exogenous factors including small molecules, growth factors, and/or genetic engineering. In some cases, transdifferentiation can be referred as direct reprogramming, which refers to the conversion of a cell type present in one tissue or organ into a cell type from another tissue or organ without going through a pluripotent cell state.

As used herein, the terms "transformed," "transduced," and "transfected" are used interchangeably unless otherwise noted. Each term refers to introduction of a nucleic acid sequence or polypeptide into a cell (e.g., an immortalized cell).

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

### 4.2. Methods for Transdifferentiating One or More Liver-Derived Cells to One or More Non-Natively Occurring Myogenic Cells Suitable for Consumption

This disclosure features methods for making non-naturally occurring myogenic cells suitable for consumption having higher total protein as compared to controls. In one non-limiting example, this disclosure features methods for transdifferentiating a liver-derived cell into a non-natively occurring myogenic cell, where the transdifferentiation results in the non-natively occurring myogenic cell having statistically significantly greater total protein content than compared to chicken fibroblasts grown in adherent conditions.

In a non-limiting example, this disclosure features methods method of making one or more non-natively occurring myogenic cells suitable for consumption where the method includes (a) contacting a population of liver-derived cells with a growth media, and (b) inducing transdifferentiation of one or more liver-derived cells into one or more non-natively occurring myogenic cells suitable for consumption by contacting the population of liver-derived cells with a differentiation media, wherein the non-natively myogenic cell comprises greater total protein (w/w%) as compared to cells not exposed to the growth media, the differentiation media, or a combination thereof. In such cases, the population of liver-derived cells are obtained (e.g., harvested) from an animal. Any appropriate means for dissection and isolation of liver-derived cells can be used. For example, obtaining the population of liver-derived cells is done using a method selected from: mechanical isolation, enzymatic digestion, and mechanical isolation and enzymatic digestion. Enzymatic digestion is performed using collagenase I, dispase, and trypsin, or combinations thereof.

In a non-limiting example, this disclosure features methods for transdifferentiating one or more liver-derived cells to one or more non-natively occurring myogenic cell suitable for consumption where the method includes (a) contacting a population of liver-derived cells with a growth media, and (b) inducing transdifferentiation of one or more liver-derived cells into one or more non-natively occurring myogenic cells suitable for consumption by contacting the population of liver-derived cells with a differentiation media, wherein the non-natively myogenic cell comprises greater total protein (w/w%) as compared to cells not exposed to the growth media, the differentiation media, or a combination thereof. In such cases, the population of liver-derived cells are obtained (e.g., harvested) from an animal. Any appropriate means for dissection and isolation of liver-derived cells can be used. For example, obtaining the population of liver-derived cells is done using a method selected from: mechanical isolation, enzymatic digestion, and mechanical isolation and enzymatic digestion. Enzymatic digestion is performed using collagenase I, dispase, and trypsin, or combinations thereof.

In another example, this disclosure features methods of making one or more non-natively occurring myogenic cells suitable for consumption, where the method includes: (a) contacting a population of liver-derived cells with a growth media, wherein the growth media comprises a modulator of Activin-A mediated signaling, a modulator of BMP-mediated signaling, a modulator of Wnt-mediated signaling, or a combination thereof; (b) adapting the population of liver-derived cells for suspension culture; and (c) inducing transdifferentiation of one or more liver-derived cells into one or more non-natively occurring myogenic cells suitable for consumption by contacting the population of liver-derived cells with a differentiation media, wherein the non-natively myogenic cell suitable for consumption comprises at least 1.5-fold greater total protein (w/w%) as compared to cells not exposed to the growth media, the suspension culture, the differentiation media, or a combination thereof. In such cases, the population of liver-derived cells are obtained (e.g., harvested) from an animal. Any appropriate means for dissection and isolation of liver-derived cells can be used. For example, obtaining the population of liver-derived cells is done using a method selected from: mechanical isolation, enzymatic digestion, and mechanical isolation and enzymatic digestion. Enzymatic digestion is performed using collagenase I, dispase, and trypsin, or combinations thereof.

In another example, this disclosure features the method for transdifferentiating one or more liver-derived cells to one or more non-natively occurring myogenic cells suitable for consumption, where the method includes: (a) contacting a population of liver-derived cells with a growth media, wherein the growth media comprises a modulator of Activin-A mediated signaling, a modulator of BMP-mediated signaling, a modulator of Wnt-mediated signaling, or a combination thereof; (b) adapting the population of liver-derived cells for suspension culture; and (c) inducing transdifferentiation of one or more liver -derived cells into one or more non-natively occurring myogenic cells suitable for consumption by contacting the population of liver-derived cells with a differentiation media, wherein the non-natively myogenic cell suitable for consumption comprises at least 1.5-fold greater total protein (w/w%) as compared to cells not exposed to the growth media, the suspension culture, the differentiation media, or a combination thereof.

In some embodiments, the method for transdifferentiating one or more liver-derived cells to one or more non-natively occurring myogenic cells suitable for consumption includes using adherent culture to expand the liver-derived cells. For example, following the obtaining from the animal, the liver-derived cells are cultured in a tissue culture flask (which can be treated) that promotes adherence of at least a portion of the liver-derived cells. In such cases, not all liver-derived cells adhere to the tissue culture flask. As such, the population of liver-derived cells can include a subpopulation of liver-derived cells that are adherent (i.e., adhered to a substrate (e.g., a tissue culture surface)) and a subpopulation of liver-derived cells that are non-adherent (e.g., liver-derived cells in suspension).

In some embodiments, the method of making (e.g., transdifferentiating) a non-natively occurring myogenic cell suitable for consumption includes adapting the liver-derived cell to be grown in suspension culture.

In some embodiments, the method of making (e.g., transdifferentiating) a non-natively occurring myogenic cell suitable for consumption includes adapting the non-natively occurring myogenic to be grown in suspension culture.

In some embodiments, the method of making (e.g., transdifferentiating) a non-natively occurring myogenic cell suitable for consumption includes adhering the population of liver-derived cells, non-natively occurring myogenic cells, or both to a cultivation infrastructure. In some embodiments, adhering liver-derived cells to the cultivation infrastructure is performed prior to contacting the population with differentiation media. In some embodiments, adhering the liver-derived cells to the cultivation infrastructure is performed in the presence differentiation medium. In such embodiments, the liver-derived cells are maintained in differentiation media for a time sufficient for production of non-natively occurring myogenic cells.

In some embodiments, the method of making (e.g., transdifferentiating) a non-natively occurring myogenic cell suitable for consumption includes culturing the liver-derived cells, the non-natively occurring myogenic cells, or both, under conditions that prevent premature differentiation. In some embodiments, these conditions include preventing nutrient deprivation that triggers premature differentiation.

In some embodiments, the method of making a non-natively occurring myogenic cell suitable for consumption includes forming a plurality of the one or more non-natively myogenic cells into a comestible (i.e., edible) food product.

In some embodiments, the transdifferentiation of a liver-derived cell to a non-natively occurring myogenic cell results in the non-natively occurring myogenic cell having higher total protein of at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to chicken fibroblasts grown in adherent conditions or myogenic cells (e.g., non-natively occurring myogenic cells) not cultured according to the methods provided herein (e.g., non-natively occurring myogenic cells not (i) contacted with the growth media; (ii) contacted with the differentiation method, (iii) adapted for cell suspension; (iv) cultured in suspension; or (v) adhered to a cultivation infrastructure). In some embodiments, the transdifferentiation of a liver-derived cell to a non-natively occurring myogenic cell results in the non-natively occurring myogenic cell having higher total protein of about 25-1000%, about 25-750%, about 25-500%, about 50-1000%, about 50-750%, about 50-500%, about 100-1000%, about 100-750%, or about 100-500%, including values and ranges therebetween, compared to chicken fibroblasts grown in adherent conditions or myogenic cells (e.g., non-natively occurring myogenic cells) not cultured according to the methods provided herein. Total protein can be measured as wet weight (e.g., w/w% BCA) or dry weight (protein % solids).

In some embodiments, the transdifferentiation of a liver-derived cell to a non-natively occurring myogenic cell results in the non-natively occurring myogenic cell having higher total protein of at least 1.025-fold, 1.05-fold, 1.10-fold, 1.15-fold, 1.20-fold, 1.25-fold, 1.30 fold, 1.35-fold, 1.40-fold, 1.45-fold, 1.5-fold, 2-fold, 2.5-fold, 3-fold, 3.5-fold, 4-fold, 4.5-fold, 5-fold, 7.5-fold, 10-fold, 15-fold, 20-fold, 25-fold, 30-fold, 40-fold, or even about 50-fold, 75-fold, 100- fold, 150-fold, or about 200-fold greater, including values and ranges therebetween, compared to chicken fibroblasts grown in adherent conditions or myogenic cells (e.g., non-natively occurring myogenic cells) not cultured according to the methods provided herein (e.g., non-natively occurring myogenic cells not (i) contacted with the growth media; (ii) contacted with the differentiation method, (iii) adapted for cell suspension; (iv) cultured in suspension; or (v) adhered to a cultivation infrastructure).

In some embodiments, the transdifferentiation of a liver-derived cell to a non-natively occurring myogenic cell results in the liver-derived cell being better suited to produce (i.e., increased differentiation capacity) cell types of interest, for example, cell types used for cultured food production (i.e., suitable for consumption), including myocytes, myoblasts, myotubes, multinucleated myotubes, satellite cells, skeletal muscle fibers, or any combination thereof. In some embodiments, the methods provided (e.g., transdifferentiation of a liver-derived cell to a non-natively occurring myogenic cell) can be used in combination with other methods for improving differentiation potential (e.g., potential to differentiate to cell types of interest including, without limitation, myocytes, myoblasts, myotubes, multinucleated myotubes, satellite cells, skeletal muscle fibers, or any combination thereof). For example, additional methods for improving differentiation potential are described in WO2015066377A1, which is herein incorporated by reference in its entirety.

In some embodiments, differentiation capacity refers to improvement in the ability of a cell line (e.g., a liver-derived cell line) to differentiate into a non-natively occurring myogenic cell as compared to a non-natively occurring myogenic cell not (i) contacted with the growth media; (ii) contacted with the differentiation method, (iii) adapted for cell suspension; (iv) cultured in suspension; and/or (v) adhered to a cultivation infrastructure. In some embodiments, the non-natively occurring myogenic cell includes myocytes, myoblasts, myotubes, multinucleated myotubes, satellite cells, skeletal muscle fibers, or any combination thereof. In some embodiments, contacting the population of liver-derived cells with a growth media results in an increase in the differentiation capacity of the liver-derived cells. In some embodiments, contacting the population of liver-derived cells with a differentiation media results in an increase in the differentiation capacity of the liver-derived cells. In some embodiments, improving the differentiation capacity of the liver-derived cells includes exposing the liver-derived cells to differentiation media comprising signaling pathway agonists, antagonist, or a combination thereof. In some embodiments, improving the differentiation capacity of the liver-derived cells includes adapting the liver-derived cells for suspension culture. In some embodiments, improving the differentiation capacity of the liver-derived cells includes culturing the liver-derived cells in suspension. In some embodiments, improving the differentiation capacity of the liver-derived cells includes adhering the liver-derived cells to a cultivation infrastructure.

In some embodiments, as a result of the methods provided herein, the liver-derived cells exhibit an increased differentiation capacity as compared to any reference strain, including other cell lines (e.g., immortalized cells lines) or primary cells.

In some embodiments, contacting the population of liver-derived cells with a growth media results in an increase in the rate of cell proliferation of the liver-derived cells. In some embodiments, contacting the population of liver-derived cells with a growth media promotes transdifferentiation of at least one liver-derived cell into a non-natively occurring myogenic cell suitable for consumption by increasing the rate of cell proliferation of the liver-derived cells. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived cells are not contacted with a growth media. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived cells are not contacted with a growth media.

In some embodiments, contacting the population of liver-derived cells with a differentiation media results in an increase in the rate of cell proliferation of the liver-derived cells. In some embodiments, contacting the population of liver-derived cells with a differentiation media promotes transdifferentiation of at least one liver-derived cell into a non-natively occurring myogenic cell suitable for consumption by increasing the rate of cell proliferation of the liver-derived cells. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived cells are not contacted with a differentiation media. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived cells are not contacted with a differentiation media.

In some embodiments, adapting the population of liver-derived cells or non-natively occurring myogenic cells for suspension culture results in an increase in the rate of cell proliferation of the liver-derived cells. In some embodiments, adapting the population of liver-derived cells or non-natively occurring myogenic cells for suspension culture promotes transdifferentiation of at least one liver-derived cell into a non-natively occurring myogenic cell suitable for consumption by increasing the rate of cell proliferation of the liver-derived cells. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived cells or non-natively occurring myogenic cells are not adapted for suspension culture. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived cells or non-natively occurring myogenic cells are not adapted for suspension culture.

In some embodiments, contacting the population of liver-derived cells with a growth media results in an increase in the cell proliferation rate of the liver-derived cells. In some embodiments, contacting the population of liver-derived cells with a growth media results in a population doubling level (PDL) (i.e., total number of times the cells in the population have doubled since their primary isolation *in vitro)* of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the liver-derived cells.

In some embodiments, contacting the population of liver-derived cells with a differentiation media results in an increase in the cell proliferation rate of the liver-derived cells. In some embodiments, contacting the population of liver-derived cells with a differentiation media enables a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the liver-derived cells.

In some embodiments, contacting the non-natively occurring myogenic cells with a differentiation media results in an increase in the cell proliferation rate of the non-natively occurring myogenic cells. In some embodiments, contacting the population of liver-derived cells with a differentiation media enables a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the non-natively occurring myogenic cells.

In some embodiments, culturing the population of liver-derived cells in suspension culture results in an increase in the cell proliferation rate of the liver-derived cells. In some embodiments, culturing the liver-derived cells in suspension culture enables population a PDL of 1, 2, 3, 4, 5, 6, 7, 8, 9, 10, 11, 12, 13, 14, 15, 16, 17, 18, 19, 20, 21, 22,23,24,25,26,27,28,29,30,31,32,33,34,35,36,37,38,39,40,41,42,43,44,45, 46, 47, 48, 49 or 50 or more passages for the liver-derived cells.

In some embodiments, culturing the non-natively occurring myogenic cells results in suspension culture having an increase in the cell proliferation rate of the non-natively occurring myogenic cells. In some embodiments, culturing the non-natively occurring myogenic cells enables population a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the liver-derived cells.

In some embodiments, prior to contacting the population of liver-derived cells with a growth media, the population of liver-derived cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, prior to contacting the population of liver-derived cells with a differentiation media, the population of liver-derived cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56

In some embodiments, prior to adopting the population of liver-derived cells for suspension culture, the population of liver-derived cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, prior to culturing the non-natively occurring myogenic cells in suspension, the population of liver-derived cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, the methods for transdifferentiating a population of liver-derived cell into a population of non-natively occurring myogenic cell results in an increased percentage of non-natively occurring myogenic cells exhibiting MyoD expression as compared to a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation comprise at least 50% (e.g., at least 55%, at least 60%, at least 65%, at least 70%, at least 75%, at least 80%, at least 85%, at least 90%, at least 95%, at least 99%, or 100%) MyoD⁺ cells.

In some embodiments, the methods for transdifferentiating a population of liver-derived cell into a population of non-natively occurring myogenic cell results in an increased percentage of non-natively occurring myogenic cells exhibiting MyHC (e.g., MyHC1) expression as compared to a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation comprise at least 50% (e.g., at least 55%, at least 60%, at least 65%, at least 70%, at least 75%, at least 80%, at least 85%, at least 90%, at least 95%, at least 99%, or 100%) MyHC⁺ cells.

In some embodiments, the methods for transdifferentiating a population of liver-derived cell into a population of non-natively occurring myogenic cell results in an increased percentage of non-natively occurring myogenic cells exhibiting Myogenin expression as compared to a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation comprise at least 50% (e.g., at least 55%, at least 60%, at least 65%, at least 70%, at least 75%, at least 80%, at least 85%, at least 90%, at least 95%, at least 99%, or 100%) Myogenin⁺ cells.

In some embodiments, the methods for transdifferentiating a population of liver-derived cell into a population of non-natively occurring myogenic cell results in increased myotube formation as compared to myotube formation resulting from a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation results in an increase in myotube formation of at least 2.5%, at least 5%, 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, as compared to myotube formation resulting from a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure.

In some embodiments, differentiation capacity of an immortalized cell line (e.g., an immortalized fibroblast cell line) is measured by determining the increase in percentage of cells that differentiate into a cell type of interest as compared to an immortalized fibroblast cell not subjected to the methods described herein. In some embodiments, differentiation capacity of an immortalized cell line is measured by determining the increase in the total number of cells that differentiate into a cell type of interest as compared to an immortalized fibroblast cell not subjected to the methods described herein. Non-limiting examples of cell types of interest (e.g., cell types produced when the immortalized cell line is exposed to differentiation media) includes: myocytes, myoblasts, myotubes, multinucleated myotubes, satellite cells, or skeletal muscle fibers, or any combination thereof.

In some embodiments, differentiation capacity of a liver-derived cell is determined by measuring paired box 7 (Pax7) expression (e.g., Pax7 RNA or protein). For example, an increase in the percentage of liver-derived cells that are Pax7⁺ as compared to a control indicates increased differentiation capacity of the liver-derived cell.

### 4.3. Method of Making a Non-Natively Occurring Myogenic Cell Suitable for Consumption Using Dedifferentiation

This disclosure also features methods for making non-naturally occurring myogenic cells suitable for consumption using dedifferentiation, where the dedifferentiation results in a non-naturally occurring myogenic cell having statistically significantly greater total protein content than compared to chicken fibroblasts grown in adherent conditions. In some embodiments, methods for making non-natively occurring myogenic cells suitable for consumption using dedifferentiation include a population of liver-derived cells dedifferentiated into dedifferentiated cells prior to undergoing differentiation to non-natively occurring myogenic cells. In such embodiments, the population of liver-derived cells are substantially free of myogenic cells. In such embodiments, the population of liver-derived cells are referred to as a population of liver-derived non-myogenic cells.

In one non-limiting example, this disclosure features methods for making a non-natively occurring myogenic cell suitable for consumption, where the method includes: (a) contacting the population of liver-derived non-myogenic cells with a growth media; (b) inducing dedifferentiation of a liver-derived cell into a dedifferentiated cell by contacting the population liver derived cells with a dedifferentiation media; and (c) inducing differentiation of the dedifferentiated cell into a non-natively occurring myogenic cell suitable for consumption by contacting the dedifferentiated cells with a differentiation media, wherein the non-natively myogenic cell comprises greater total protein (w/w%) as compared to cells not exposed to the growth media, the suspension culture, the differentiation media, or a combination thereof. In some embodiments, step (b) comprises contacting the population of liver-derived cells with a dedifferentiation media (e.g., any of the dedifferentiation medias described herein). In some embodiments, step (c) comprises contacting the dedifferentiated cells with a differentiation media. In such embodiments, the population of liver-derived cells are obtained (e.g., harvested) from an animal. Any appropriate means for dissection and isolation of liver-derived cells can be used. For example, obtaining the population of liver-derived cells is done using a method selected from: mechanical isolation, enzymatic digestion, or mechanical isolation and enzymatic digestion. In such embodiments, enzymatic digestion is performed using collagenase I, dispase, and trypsin, or combinations thereof.

In another non-limiting example, this disclosure features a method for making a non-natively occurring myogenic cell suitable for consumption where the method includes: (a) contacting a population of liver-derived non-myogenic cells with a growth media; (b) contacting the population of liver-derived non-myogenic cells with a dedifferentiation media under conditions sufficient to induce dedifferentiation of at least one liver-derived cell into a dedifferentiated cell; and (c) contacting the dedifferentiated cells with a differentiation media under conditions sufficient to induce differentiation of the dedifferentiated cell into a non-natively occurring myogenic cell, thereby producing a non-natively occurring myogenic cell suitable for consumption. In such embodiments, the population of liver-derived cells are obtained (e.g., harvested) from an animal using any appropriate means for dissection and isolation known in the art or described herein.

In some embodiments, the method of making non-natively occurring myogenic cells based on dedifferentiation includes using adherent culture to expand the liver-derived non-myogenic cells. For example, following the obtaining from the animal, the liver-derived cells are cultured in a tissue culture flask that promotes adherence of at least a portion of the liver-derived non-myogenic cells. In such cases, not all liver-derived non-myogenic cells adhere to the tissue culture flask. As such, the population of liver-derived non-myogenic cells can include a subpopulation of liver-derived non-myogenic cells that are adherent (i.e., adhered to a substrate (e.g., a tissue culture surface)) and a subpopulation of liver-derived non-myogenic cells that are non-adherent (e.g., liver-derived cells growing in suspension).

In some embodiments, the method of making non-natively occurring myogenic cells using dedifferentiation includes adapting the population of liver-derived non-myogenic cells for suspension culture.

In some embodiments, the method of making non-natively occurring myogenic cells using dedifferentiation includes adapting the non-natively occurring myogenic cells for suspension culture.

In some embodiments, the method of making non-natively occurring myogenic cells using dedifferentiation includes adhering the population of liver-derived non-myogenic cells, dedifferentiated cells, non-natively occurring myogenic cells, or any combination thereof to a cultivation infrastructure. In some embodiments, adhering liver-derived cells to the cultivation infrastructure is performed prior to contacting the population with differentiation media. In some embodiments, adhering the liver-derived cells to the cultivation infrastructure is performed in the presence dedifferentiation medium. In such embodiments, the liver-derived cells are maintained in dedifferentiation media for a time sufficient for production of dedifferentiated cells. In some embodiments, adhering dedifferentiated cells to the cultivation infrastructure is performed prior to contacting the population of dedifferentiated cells with differentiation media. In some embodiments, adhering the dedifferentiated cells to the cultivation infrastructure is performed in the presence differentiation medium. In such embodiments, the dedifferentiated cells are maintained in differentiation media for a time sufficient for production of non-natively occurring myogenic cells.

In some embodiments, the method of making a non-natively occurring myogenic cell using dedifferentiation (e.g., inducing dedifferentiation of the liver-derived non-myogenic cell to a dedifferentiated cell and differentiation of the dedifferentiated cell to a non-natively occurring myogenic cell) results in the non-natively occurring myogenic cell having higher total protein at least 2.5%, at least 5%, 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to tissue generated with chicken fibroblasts grown in adherent conditions or myogenic cells (e.g., non-natively occurring myogenic cells) not cultured according to the methods provided herein. In some embodiments, the method of making a non-natively occurring myogenic cell using dedifferentiation (e.g., inducing dedifferentiation of the liver-derived non-myogenic cell to a dedifferentiated cells and differentiation of the dedifferentiated cell to a non-natively occurring myogenic cell) results in the non-natively occurring myogenic cell having higher total protein about 25-1000%, about 25-750%, about 25-500%, about 50-1000%, about 50-750%, about 50-500%, about 100-1000%, about 100-750%, or about 100-500%, including values and ranges therebetween, compared to tissue generated with chicken fibroblasts grown in adherent conditions or myogenic cells (e.g., non-natively occurring myogenic cells) not cultured according to the methods provided herein. Total protein can be measured as wet weight (e.g., w/w% BCA) or dry weight (protein % solids).

In some embodiments, the method of making a non-natively occurring myogenic cell using dedifferentiation (e.g., inducing dedifferentiation of the liver-derived non-myogenic cell to a dedifferentiated cell and differentiation of the dedifferentiated cell to a non-natively occurring myogenic cell) results in the non-natively occurring myogenic cell having higher total protein at least at least 1.025-fold, 1.05-fold, 1.10-fold, 1.15-fold, 1.20-fold, 1.25-fold, 1.30 fold, 1.35-fold, 1.40-fold, 1.45-fold, 1.5-fold, 2-fold, 2.5-fold, 3-fold, 3.5-fold, 4-fold, 4.5-fold, 5-fold, 7.5-fold, 10-fold, 15-fold, 20-fold, 25-fold, 30-fold, 40-fold, or even about 50-fold, 75-fold, 100- fold, 150-fold, or about 200-fold greater, including values and ranges therebetween, compared to tissue generated with chicken fibroblasts grown in adherent conditions or myogenic cells (e.g., non-natively occurring myogenic cells) not cultured according to the methods provided herein.

In some embodiments, the method of making a non-natively occurring myogenic cell using dedifferentiation (e.g., inducing dedifferentiation of the liver-derived non-myogenic cell to a dedifferentiated cells and differentiation of the dedifferentiated cell to a non-natively occurring myogenic cell) results in the liver-derived cell being better suited to produce (i.e., increased differentiation capacity) cell types of interest, for example, cell types used for cultured food production, including myocytes, myoblasts, myotubes, multinucleated myotubes, satellite cells, skeletal muscle fibers, or any combination thereof. In some embodiments, the methods provided (e.g., inducing dedifferentiation of the liver-derived non-myogenic cell to a dedifferentiated cells and inducing differentiation of the dedifferentiated cell to a non-natively occurring myogenic cell)) can be used in combination with other methods for improving differentiation potential. For example, additional methods for improving differentiation potential are described in WO2015066377A1, which is herein incorporated by reference in its entirety.

In some embodiments, differentiation capacity refers to improvement in the ability of a cell line (e.g., a liver-derived cell line) to differentiate into a non-natively occurring myogenic cell as compared to a myogenic cell (e.g., a non-natively occurring myogenic cell) not (i) contacted with the growth media; (ii) contacted with the differentiation method, (iii) adapted for cell suspension; (iv) cultured in suspension; and/or (v) adhered to a cultivation infrastructure. In some embodiments, the non-natively occurring myogenic cell includes myocytes, myoblasts, myotubes, multinucleated myotubes, satellite cells, skeletal muscle fibers, or any combination thereof. In some embodiments, contacting the population of liver-derived non-myogenic cells with a growth media results in an increase in the differentiation capacity of the liver-derived cells. In some embodiments, contacting the population of liver-derived non-myogenic cells with a differentiation media results in an increase in the differentiation capacity of the liver-derived non-myogenic cells. In some embodiments, contacting the population of liver-derived non-myogenic cells with a dedifferentiation media results in an increase in the differentiation capacity of the liver-derived non-myogenic cells. In some embodiments, improving the differentiation capacity of the liver-derived cells includes exposing the liver-derived cells to differentiation (or dedifferentiation) media comprising signaling pathway agonists, antagonist, or a combination thereof. In some embodiments, improving the differentiation capacity of the liver-derived non-myogenic cells includes adapting the liver-derived cells for suspension culture. In some embodiments, improving the differentiation capacity of the liver-derived non-myogenic cells includes culturing the liver-derived non-myogenic cells in suspension. In some embodiments, improving the differentiation capacity of the liver-derived non-myogenic cells includes adhering the population of liver-derived non-myogenic cells to a cultivation infrastructure. In some embodiments, improving the differentiation capacity of the liver-derived non-myogenic cells includes dedifferentiating the liver-derived non-myogenic cells into dedifferentiated cells.

In some embodiments, contacting the population of liver-derived cells with a growth media promotes dedifferentiation of at least one liver-derived cell into a dedifferentiated cell by increasing the rate of cell proliferation of the liver-derived non-myogenic cells. In some embodiments, increase in the rate of cell proliferation is at least 2.5%, at least 5%, 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived non-myogenic cells are not contacted with a growth media. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived non-myogenic cells are not contacted with a growth media.

In some embodiments, contacting the population of liver-derived non-myogenic cells with a dedifferentiation media results in an increase in the rate of cell proliferation of the liver-derived cells or dedifferentiated cells, respectively. In some embodiments, contacting the population of liver-derived non-myogenic cells with a dedifferentiation media promotes dedifferentiation of at least one liver-derived non-myogenic cell into a dedifferentiated cell by increasing the rate of cell proliferation of the liver-derived non-myogenic cells. In some embodiments, contacting the population of liver-derived non-myogenic cells or dedifferentiated cells with a dedifferentiation media promotes dedifferentiation of at least one liver-derived non-myogenic cell into dedifferentiated cell by increasing the rate of cell proliferation of the liver-derived non-myogenic cell or dedifferentiated cells. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived non-myogenic cells or dedifferentiated cells are not contacted with a dedifferentiation media. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived non-myogenic cells or dedifferentiated cells are not contacted with a dedifferentiation media.

In some embodiments, contacting the population of liver-derived non-myogenic cells with a differentiation media results in an increase in the rate of cell proliferation of the liver-derived cells or dedifferentiated cells, respectively. In some embodiments, contacting the population of liver-derived non-myogenic cells or dedifferentiated cells with a differentiation media promotes differentiation of at least one liver-derived non-myogenic cell or dedifferentiated cell into a non-natively occurring myogenic cell. In some embodiments, contacting the population of liver-derived non-myogenic cells with a differentiation media promotes differentiation of at least one liver-derived non-myogenic cell or dedifferentiated cell into a non-natively occurring myogenic cell suitable for consumption by increasing the rate of cell proliferation of the liver-derived non-myogenic cell or dedifferentiated cells. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived non-myogenic cells or dedifferentiated cells are not contacted with a differentiation media. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived non-myogenic cells or dedifferentiated cells are not contacted with a differentiation media.

In some embodiments, adapting the population of liver-derived non-myogenic cells, dedifferentiated cells, or non-natively occurring myogenic cells for suspension culture results in an increase in the rate of cell proliferation of the liver-derived non-myogenic cells, dedifferentiated cells, or non-natively occurring myogenic cells, respectively. In some embodiments, adapting the population of liver-derived non-myogenic cells or dedifferentiated cells for suspension culture promotes transdifferentiation of at least one liver-derived cell into a non-natively occurring myogenic cell suitable for consumption by increasing the rate of cell proliferation of the liver-derived cells. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived non-myogenic cells or dedifferentiated cells are not adapted for suspension culture. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived non-myogenic cells or non-natively occurring myogenic cells are not adapted for suspension culture.

In some embodiments, contacting the population of liver-derived non-myogenic cells with a growth media results in an increase in the cell proliferation rate of the liver-derived non-myogenic cells. In some embodiments, contacting the population of liver-derived non-myogenic cells with a growth media enables a population doubling level (PDL) (i.e., total number of times the cells in the population have doubled since their primary isolation *in vitro)* of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the liver-derived cells.

In some embodiments, contacting the population of liver-derived non-myogenic cells with a dedifferentiation media results in an increase in the cell proliferation rate of the liver-derived non-myogenic cells or dedifferentiated cells. In some embodiments, contacting the population of liver-derived non-myogenic cells with a dedifferentiation media enables a PDL of 1, 2, 3, 4, 5, 6, 7, 8, 9, 10, 11, 12, 13, 14, 15, 16, 17, 18, 19, 20, 21,22,23,24,25,26,27,28,29,30,31,32,33,34,35,36,37,38,39,40,41,42,43,44, 45, 46, 47, 48, 49 or 50 or more passages for the liver-derived non-myogenic cells or dedifferentiated cells.

In some embodiments, contacting the population of liver-derived non-myogenic cells with a differentiation media results in an increase in the cell proliferation rate of the liver-derived non-myogenic cells or dedifferentiated cells. In some embodiments, contacting the population of liver-derived non-myogenic cells with a differentiation media enables a PDL of 1, 2, 3, 4, 5, 6, 7, 8, 9, 10, 11, 12, 13, 14, 15, 16, 17, 18, 19, 20, 21, 22, 23, 24, 25, 26,27,28,29,30,31,32,33,34,35,36,37,38,39,40,41,42,43,44,45,46,47,48,49 or 50 or more passages for the liver-derived non-myogenic cells or dedifferentiated cells.

In some embodiments, culturing the liver-derived non-myogenic cells in suspension culture results in an increase in the cell proliferation rate of the liver-derived non-myogenic cells. In some embodiments, culturing the liver-derived cells in suspension culture enables a PDL of 1, 2, 3, 4, 5, 6, 7, 8, 9, 10, 11, 12, 13, 14, 15, 16, 17, 18, 19, 20, 21,22,23,24,25,26,27,28,29,30,31,32,33,34,35,36,37,38,39,40,41,42,43,44, 45, 46, 47, 48, 49 or 50 or more passages for the liver-derived non-myogenic cells.

In some embodiments, dedifferentiation of the liver-derived non-myogenic cells to form dedifferentiated cells results in an increase in the cell proliferation rate of the liver-derived non-myogenic cells and/or the dedifferentiated cells. In some embodiments, dedifferentiation of the liver-derived non-myogenic cells to form dedifferentiated cells enables a PDL of 1, 2, 3, 4, 5, 6, 7, 8, 9, 10, 11, 12, 13, 14, 15, 16, 17, 18, 19, 20, 21, 22, 23,24,25,26,27,28,29,30,31,32,33,34,35,36,37,38,39,40,41,42,43,44,45,46, 47, 48, 49 or 50 or more passages for the liver-derived non-myogenic cells and/or the dedifferentiated cells.

In some embodiments, prior to contacting the population of liver-derived non-myogenic cells with a growth media, the population of liver-derived cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, prior to contacting the population of liver-derived cells with a dedifferentiation media, the population of liver-derived cells or dedifferentiated cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, prior to contacting the population of liver-derived cells with a differentiation media, the population of liver-derived cells or dedifferentiated cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, prior to adopting the population of liver-derived cells for suspension culture, the population of liver-derived cells or dedifferentiated cells include less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, prior to culturing the non-natively occurring myogenic cells in suspension, the population of non-natively occurring myogenic cells includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, the method of making a non-natively occurring myogenic cell using dedifferentiation (e.g., inducing dedifferentiation of the liver-derived non-myogenic cell to a dedifferentiated cell and differentiation of the dedifferentiated cell to a non-natively occurring myogenic cell) results in an increased percentage of non-natively occurring myogenic cells exhibiting MyoD expression as compared to a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation comprise at least 50% (e.g., at least 55%, at least 60%, at least 65%, at least 70%, at least 75%, at least 80%, at least 85%, at least 90%, at least 95%, at least 99%, or 100%) MyoD⁺ cells.

In some embodiments, the method of making a non-natively occurring myogenic cell using dedifferentiation (e.g., inducing dedifferentiation of the liver-derived non-myogenic cell to a dedifferentiated cell and differentiation of the dedifferentiated cell to a non-natively occurring myogenic cell) results in an increased percentage of non-natively occurring myogenic cells exhibiting MyHC1 expression as compared to a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation comprise at least 50% (e.g., at least 55%, at least 60%, at least 65%, at least 70%, at least 75%, at least 80%, at least 85%, at least 90%, at least 95%, at least 99%, or 100%) MyHC⁺ cells.

In some embodiments, the method of making a non-natively occurring myogenic cell using dedifferentiation (e.g., inducing dedifferentiation of the liver-derived non-myogenic cell to a dedifferentiated cell and differentiation of the dedifferentiated cell to a non-natively occurring myogenic cell) results in an increased percentage of non-natively occurring myogenic cells exhibiting myogenin expression as compared to a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the transdifferentiation comprise at least 50% (e.g., at least 55%, at least 60%, at least 65%, at least 70%, at least 75%, at least 80%, at least 85%, at least 90%, at least 95%, at least 99%, or 100%) Myogenin⁺ cells.

In some embodiments, the method of making a non-natively occurring myogenic cell using dedifferentiation (e.g., inducing dedifferentiation of the liver-derived non-myogenic cell to a dedifferentiated cell and differentiation of the dedifferentiated cell to a non-natively occurring myogenic cell) results in increased myotube formation as compared to myotube formation resulting from a population of non-natively occurring myogenic cells not (i) contacted with the growth media; (ii) contacted with the differentiation method, (iii) adapted for cell suspension; (iv) cultured in suspension; and/or (v) adhered to a cultivation infrastructure. In some embodiments, the population of non-natively occurring myogenic cells produced as a result of the dedifferentiation (and subsequent differentiation) results in an increase in myotube formation of at least 2.5%, at least 5%, 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, as compared to myotube formation resulting from a population of myogenic cells (e.g., non-natively occurring myogenic cells) not (i) contacted with the growth media; (ii) contacted with the differentiation method, (iii) adapted for cell suspension; (iv) cultured in suspension; and/or (v) adhered to a cultivation infrastructure.

### 4.4. Method for Improving Anchorage Independent Growth using Transdifferentiation or Dedifferentiation of Liver-Derived cells to a Non-Natively Occurring Myogenic Cell

In some embodiments, the methods provided herein promote/enhance anchorage-independent growth of the liver-derived cells.

In some embodiments, contacting the population of liver-derived cells with a growth media results in an increase in the rate of cell proliferation and decrease in cell death, which promotes adherent cells to transition to a non-adherent form. In some embodiments, an increase in the rate of cell proliferation and decrease in cell death promotes transition of anchorage-independent growth from anchorage-dependent growth.

In some embodiments, contacting the population of liver-derived cells with a dedifferentiation media or differentiation media results in an increase in the rate of cell proliferation and decrease in cell death, which promotes adherent cells to transition to a non-adherent form. In some embodiments, an increase in the rate of cell proliferation and decrease in cell death promotes transition of anchorage-independent growth from anchorage-dependent growth.

In some embodiments, adapting the population of liver-derived cells, dedifferentiated cells, or non-natively occurring myogenic cells for suspension culture results in an increase in the rate of cell proliferation of the liver-derived cells, dedifferentiated cells, or non-natively occurring myogenic cells, which promotes adherent cells to transition to a non-adherent form. In some embodiments, an increase in the rate of cell proliferation and decrease in cell death promotes transition of anchorage-independent growth from anchorage-dependent growth.

The rate of cell proliferation can be assessed, for example, by counting the number of cells in the S-phase. In some embodiments, increase in the rate of cell proliferation is at least 2.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 150%, at least 200%, at least 250%, at least 300%, at least 350%, at least 400%, at least 450%, at least 500%, at least 550%, at least 600%, at least 650%, at least 700%, at least 750%, at least 800%, at least 850%, at least 900%, at least 950%, at least 1000%, including values and ranges therebetween, compared to when the population of liver-derived cells, dedifferentiated cells, or non-natively occurring myogenic cells are not cultured according to the methods described herein. In some embodiments, the increase in the rate of cell proliferation is about 25-1000%, about 25-750%, about 25-500%, about 50-1000%, about 50-750%, about 50-500%, about 100-1000%, about 100-750%, or about 100-500%, including values and ranges therebetween, compared to when the population of liver-derived cells, dedifferentiated cells, or non-natively occurring myogenic cells are not cultured according to the methods described herein.

In some embodiments, the methods of transdifferentiating or dedifferentiating as described herein promote anchorage-independent growth by decreasing cell-to-cell contact inhibition. In some embodiments, the decrease in contact inhibition provided by the present methods is about 10%, 20%, 30%, 40%, 50%, 60%, 70%, 80%, 90%, or 100%, including values and ranges therebetween, compared to when the population of liver-derived cells, dedifferentiated cells, or non-natively occurring myogenic cells are not cultured according to the methods described herein.

In some embodiments, the methods of transdifferentiating or dedifferentiating as described herein promote anchorage-independent growth by decreasing cell death. In some embodiments, the decrease in contact inhibition provided by the present methods is about 2.5%, 5%, 10%, 20%, 30%, 40%, 50%, 60%, 70%, 80%, 90%, or 100%, including values and ranges therebetween, compared to when the population of liver-derived cells, dedifferentiated cells, or non-natively occurring myogenic cells are not cultured according to the methods described herein.

### 4.5. Obtaining Liver-Derived Cells

This disclosure features methods of making a non-natively occurring myogenic cell suitable for consumption by first obtaining a cell or population of cells from a liver. As used herein, "liver" refers to the cells, cellular compartments, and architecture of a liver, for example, as described in Si-Tayeb et al. (Developmental Cell, 18(2): 175-189 (2010)), which is herein incorporated by reference in its entirety. In some embodiments, the population of liver-derived cells is substantially free of myogenic cells (e.g., non-muscle liver-derived cells or liver-derived non-muscle cells).

In some embodiments, the non-muscle liver-derived cells are obtained (e.g., harvested) from embryonic liver, fetal liver, or neo-natal liver. In some embodiments, the non-muscle-liver derived cells are obtained during liver bud growth, hepatocyte differentiation, and/or hepatic maturation.

In some embodiments, the non-muscle liver-derived cells are obtained (e.g., harvested) from an animal (e.g., any of the animals described herein or known in the art) having an age at about embryonic day 1 (E1), about E2, about E3, about E4, about E5, about E6, about E7, about E8, about E9, about E10, about E11, about E12, about E13, about E14, about E15, about E16, about E17, about E18, about E19, about E20, about E21, about E22, about E23, about E24, about E25, about E26, about E27, about E28, about E29, or about E30.

In some embodiments, the non-muscle liver-derived cells are obtained (e.g., harvested) from an animal (e.g., any of the animals described herein or known in the art) at about 1 week of gestation, about 2 weeks of gestation, about 3 weeks of gestation, about 4 weeks of gestation, about 5 weeks of gestation, about 6 weeks of gestation, about 7 weeks of gestation, about 8 weeks of gestation, about 9 weeks of gestation, about 10 weeks of gestation, about 11 weeks of gestation, about 12 weeks of gestation, about 13 weeks of gestation, about 14 weeks of gestation, about 15 weeks of gestation, about 16 weeks of gestation, about 17 weeks of gestation, about 18 weeks of gestation, about 19 weeks of gestation, or about 20 or more weeks of gestation.

In some embodiments, the non-muscle liver-derived cells are obtained (e.g., harvested) from an animal (e.g., any of the animals described herein or known in the art) at about 1 week after birth, about 2 weeks after birth, about 3 weeks after birth, about 4 weeks after birth, about 5 weeks after birth, about 6 weeks after birth, about 7 weeks after birth, about 8 weeks after birth, about 9 weeks after birth, about 10 weeks after birth, about 11 weeks after birth, about 12 weeks after birth, about 13 weeks after birth, about 14 weeks after birth, about 15 weeks after birth, or about 16 or more weeks after birth.

In some embodiments, the liver-derived cells are obtained (e.g., harvested) from liver (e.g., embryonic liver) that include myogenic precursor cells. In some embodiments, the population of liver-derived cells contacted with culture media (e.g., growth media) is substantially free of liver-derived myogenic precursor cells.

In some embodiments, the population of liver-derived cells obtained from the animal includes less than 10% (e.g., less than 9%, less than 8%, less than 7%, less than 6%, less than 5%, less than 4%, less than 3%, less than 2%, less than 1%, less than 0.9%, less than 0.8%, less than 0.7%, less than 0.6%, less than 0.5%, less than 0.4%, less than 0.3%, less than 0.2%, or less than 0.1%) of cells expressing any one or more of Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, the population of liver-derived cells contacted with culture media (e.g., growth media) includes liver-derived myogenic precursor cells. In some embodiments, a myogenic precursor cells express one or more of the markers selected from: Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56. In some embodiments, the population of liver-derived cells includes at least 5% *(*e.g., at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, least 31%, at least 32%, at least 33%, at least 34%, at least 35%, at least 36%, at least 37%, at least 38%, at least 39%, at least 40%, least 41%, at least 42%, at least 43%, at least 44%, at least 45%, at least 46%, at least 47%, at least 48%, at least 49%, at least 50%, least 51%, at least 52%, at least 53%, at least 54%, at least 55%, at least 56%, at least 57%, at least 58%, at least 59%, at least 60%, least 61%, at least 62%, at least 63%, at least 64%, at least 65%, at least 66%, at least 67%, at least 68%, at least 69%, at least 70%, least 71%, at least 72%, at least 73%, at least 74%, at least 75%, at least 76%, at least 77%, at least 78%, at least 79%, at least 80%, least 81%, at least 82%, at least 83%, at least 84%, at least 85%, at least 86%, at least 87%, at least 88%, at least 89%, at least 90%, least 99%, at least 92%, at least 93%, at least 94%, at least 95%, at least 96%, at least 97%, at least 98%, or at least 99%) of cells expressing one or more of the markers selected from: Pax7, Pax3, MyoD, ABCG2, M-Cadherin/Cadherin-15, Caveolin-1, CD34, FoxK1, Integrin alpha 7, Integrin alpha 7 beta 1, MYF-5, Myogenin, and NCAM-1/CD56.

In some embodiments, the non-muscle liver-derived cells are obtained (e.g., harvested) from liver (e.g., embryonic liver) that include myofibroblasts (e.g., myofibroblasts having exocytotic vesicles, stress fibers, and/or smooth muscle actin staining). In such cases, the population of liver-derived cells contacted with culture media (e.g., growth media) include myofibroblasts.

In some embodiments, the non-muscle liver-derived cells are obtained (e.g., harvested) from liver (e.g., embryonic liver) that include hepatic stem cells (e.g., a hepatic stem cell expresses one or more of the markers selected from: Sox9, Fox1, Lgr5, Pkh2, CD133, CD29, EpCAM, and E-cadherin). In such cases, the population of liver-derived cells contacted with culture media (e.g., growth media) include hepatic stem cells.

In some embodiments, the population of liver-derived cells is substantially free of hepatic stem cells. In some embodiments, the methods provided herein include a step of separating out hepatic stem cells prior to contacting the cells with a culture media (e.g., a growth media). In some embodiments, a hepatic stem cell expresses one or more of the markers selected from: Sox9, FoxL1, Lgr5, Pkh2, CD133, CD29, EpCAM, and E-cadherin.

In some embodiments, the population of liver-derived cells contacted with culture media is substantially free of cells expressing one or more liver cell markers. A non-limiting example of identifying and isolating hepatic cells is as described in U.S. Pat. No. 8,691,523, which is herein incorporated by reference in its entirety.

In some embodiments, the population of liver-derived cells is substantially free of cells that have undergone senescence.

In some embodiments, the population of liver-derived cells is substantially free of cells that have acquired mutations that increase cell proliferation rates (e.g., mutations that confer a growth advantage over other cells in the culture).

In some embodiments, the population of liver-derived cells do not acquire mutations after contacting the culture media (e.g., growth media, dedifferentiation media, or differentiation media).

In some embodiments, the population of liver-derived cells is substantially free of differentiated cells. In some embodiments, the population of liver-derived cells is substantially free of terminally differentiated cells.

In some embodiments, the population of liver-derived cells is substantially free of multipotent stem cells. Multipotent stem cells include, without limitation, myosatellite cells, hepatoblasts, mesenchymal stem cells, and other progenitor cells.

In some embodiments, the population of liver-derived cells is substantially free of hematopoietic cells. In some embodiments, the methods provided herein include a step of separating out hematopoietic cells prior to contacting the liver-derived cells with a culture media (e.g., a growth media, a dedifferentiation media, or a differentiation media). A non-limiting example of separating out hematopoietic cells is as described in U.S. Pat. No. 8,691,523, which is herein incorporated by reference in its entirety.

Methods of obtaining (e.g., harvesting) a liver-derived cell or population of liver-derived cells are known in the art, for example, without limitation, U.S. Pat. No. 8,691,523, which is herein incorporated by reference in its entirety.

In some embodiments, the non-muscle liver-derived cells are obtained (e.g., harvested) from livestock such as domestic cattle, pigs, sheep, goats, camels, water buffalo, rabbits and the like. In some embodiments, the non-muscle liver-derived cells are obtained (e.g., harvested) from poultry, for example, without limitation, domesticated poultry, such as chicken, turkeys, ducks, geese, and pigeons. In some embodiments, the non-muscle liver-derived cells are from game species, for example, without limitation, wild deer, gallinaceous fowl, waterfowl, and hare. In some embodiments, the non-muscle liver-derived cells are obtained (e.g., harvested) from aquatic species or semi-aquatic species, including, without limitation. certain fish, crustaceans, mollusks, cephalopods, cetaceans, crocodilians, turtles, and frogs. In some embodiments, the non-muscle liver-derived cells are from exotic, endangered, conserved or extinct animal species.

In some embodiments, the non-muscle liver-derived cells are obtained (e.g., harvested) from *Gallus, Bos taurus, Sous scrofa*, *Meleagris gallopavo*, *Anas platyrynchos, Salmo salar*, *Thunnus thynnus, Ovis aries, Coturnix, Capra aegagrus hircus,* or *Homarus americanus.*

In an exemplary embodiment, the non-muscle liver-derived cells are obtained (e.g., harvested) from livestock, poultry, game, or aquatic species.

In some embodiments, the liver-derived cells are not natively myogenic (e.g. are non-myogenic cells such as fibroblasts or non-myogenic stem cells that are cultured to become myogenic cells in the cultivation infrastructure).

In some embodiments, the liver-derived cells are somatic cells. In some embodiments, the liver-derived cells are not somatic cells.

In some embodiments, the non-natively occurring myogenic cells are of the skeletal muscle lineage. Cells of the skeletal muscle lineage include myoblasts, myocytes, and skeletal muscle progenitor cells, also called myogenic progenitors, that include satellite cells, side population cells, muscle derived stem cells, mesenchymal stem cells, myogenic pericytes, and mesoangioblasts.

In some embodiments, the liver-derived cells, dedifferentiated cells, or non-natively occurring myogenic cells are cultivated in a suspension culture and form a self-adherent aggregate. A self-adherent aggregate refers to masses of viable cells suspended in a physiological liquid medium (e.g., suspension culture) aggregated due to, for example, their (1) adherence to each other (e.g., cadherin cell adhesion) (2) adherence to a basement membrane or other extracellular matrix secreted by the cells (e.g., integrin cell adhesion) or (3) a combination of both.

In some embodiments, the non-muscle liver-derived cells are obtained (e.g., harvested) for the formulation of cell-based food products, such as cultured animal meat. The term "cultured meat" as used herein refers to uncooked or cooked meat produced using cell culture methods. In some embodiments, the methods utilize cells with the potential to differentiate into skeletal muscle.

### 4.6. Culture Media

This disclosure features methods of making a non-natively occurring myogenic cell suitable for consumption by obtaining a liver-derived cell and culturing the liver-derived cell in culture media, including a growth media, dedifferentiation media, and/or a differentiation media. For example, a method for making a non-natively occurring myogenic cell suitable for consumption comprises exposing the liver-derived cell to at least one of a growth media, a dedifferentiation media, a differentiation media, or any combination or order thereof.

In some embodiments, non-natively myogenic cells suitable for consumption are grown in edible nutrient medium as described in U.S. Patent Publication No. 2022/0073870, which is herein incorporated by reference in its entirety.

### 4.6.1. Growth Media

In some embodiments, a growth media comprises base media without any additional additives. Non-limiting examples of base media include: DMEM/F12, MEM, and IMDM. In some embodiments, a growth media comprises base media including serum. For example, growth media includes about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19%, or about 20% serum. In some embodiments, differentiation media includes about 20% serum. In some embodiments, differentiation media includes about 10% serum. In some embodiments, the growth media is serum free.

In some embodiments, growth media includes serum derived from two or more species. In some embodiments, serum is selected from; fetal bovine serum (FBS), chicken serum, and horse serum, or a combination thereof. In some embodiments, growth media includes FBS and horse serum, FBS and chicken serum, horse serum and chicken serum, or FBS, chicken serum, and horse serum. In some embodiments, growth media includes about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19% or about 20% of a first serum (e.g., FBS) and about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19% or about 20% of a second serum (e.g., chicken serum).

In some embodiments, growth media includes signaling pathway modulators. In some embodiments, the signaling pathways can either be activated or inhibited. For example, without limitation, Wnt, TGF (Activin A), and BMP signaling pathways can be activated (e.g., using CHIR99021, Activin A, or BMP4, respectively) or inhibited (e.g., using IWR1, A-83-01, or LDN193189, respectively). In some embodiments, growth media includes an activator of Wnt signaling (e.g., CHIR99021), an inhibitor of TGF (Activin A) signaling (e.g., A-83-01), and an inhibitor of BMP signaling (e.g., LDN193189).

In some embodiments, the methods described herein can include culture media as described in U.S. Application Serial No. 63/293,578 (filed on December 23, 2021).

In some embodiments, growth media is selected from: (i) DMEM/F12, about 20% FBS, and about 5% chicken serum; (ii) DMEM/F12, about 20% FBS, about 5% chicken serum, CHIR99021, A-83-01, and LDN193189; and (iii) DMEM/F12, about 10% FBS, and about 5% chicken serum.

In some embodiments, the methods provided herein include contacting a liver-derived cell (e.g., a population of liver-derived cells) with a growth media. In such cases, the liver-derived cells (e.g., the population of liver-derived cells) are exposed to the growth media for a first period of time. As used herein the phrase "a first period of time" includes any period of time sufficient to allow for expansion of the liver-derived cells (e.g., the population of liver-derived cells) to quantities that allow for production of non-natively occurring myogenic cells suitable for consumption. For example, a first period of time includes from about day 1 to about day 14 (e.g., from about day 1 to about day 13, from about day 1 to about day 12, from about day 1 to about day 11, from about day 1 to about day 10, from about day 1 to about day 9, from about day 1 to about day 8, from about day 1 to about day 7, from about day 1 to about day 6, from about day 1 to about day 5, from about day 1 to about day 4, from about day 2 to about day 14, from about day 2 to about day 13, from about day 2 to about day 12, from about day 2 to about day 11, from about day 2 to about day 10, from about day 2 to about day 9, from about day 2 to about day 8, from about day 2 to about day 7, from about day 2 to about day 6, from about day 2 to about day 5, from about day 2 to about day 4, from about day 3 to about day 14, from about day 3 to about day 13, from about day 3 to about day 12, from about day 3 to about day 11, from about day 3 to about day 10, from about day 3 to about day 9, from about day 3 to about day 8, from about day 3 to about day 7, from about day 3 to about day 6, from about day 3 to about day 5, from about day 3 to about day 4, from about day 4 to about day 14, from about day 4 to about day 13, from about day 4 to about day 12, from about day 4 to about day 11, from about day 4 to about day 10, from about day 4 to about day 9, from about day 4 to about day 8, from about day 4 to about day 7, from about day 4 to about day 6, from about day 4 to about day 5, from about day 5 to about day 14, from about day 5 to about day 14, from about day 5 to about day 13, from about day 5 to about day 12, from about day 5 to about day 11, from about day 5 to about day 10, from about day 5 to about day 9, from about day 5 to about day 8, from about day 5 to about day 7, from about day 5 to about day 6, from about day 6 to about day 14, from about day 6 to about day 13, from about day 6 to about day 12, from about day 6 to about day 11, from about day 6 to about day 10, from about day 6 to about day 9, from about day 6 to about day 8, from about day 6 to about day 7, from about day 7 to about day 13, from about day 7 to about day 12, from about day 7 to about day 11, from about day 7 to about day 10, from about day 8 to about day 13, from about day 8 to about day 12, from about day 8 to about day 10, from about day 9 to about day 13, from about day 9 to about day 12, from about day 9 to about day 11, from about day 10 to about day 13, or from about day 11 to about day 13) of culture, where day 0 is the day the liver-derived cells are first contacted with the growth media.

In some embodiments, a first period of time includes 10, 11, 12, 13, 14, 15, 16, 17, 18, 19, 20, 21, 22, 23, 24, 25, 26, 27, 28, 29 or 30 or more days. In some embodiments, a first period of time includes 1 month, 2 months, 3 months, 4 months, 5 months, 6 months, 7 months, 8 months, 9 months, 10 months, 11 months or 12 months or more.

In some embodiments, a first period of time includes the amount of time needed for the population of liver-derived cells to have a population doubling level (PDL) (i.e., total number of times the cells in the population have doubled since their primary isolation *in vitro)* of 1, 2, 3, 4, 5, 6, 7, 8, 9, 10, 11, 12, 13, 14, 15, 16, 17, 18, 19, 20, 21, 22, 23, 24, 25, 26,27,28,29,30,31,32,33,34,35,36,37,38,39,40,41,42,43,44,45,46,47,48,49 or 50 or more passages.

In some embodiments, the methods provided herein include contacting a liver-derived cell (e.g., a population of liver-derived cells) with two or more different growth medias.

In some embodiments, the step of contacting the liver-derived cells (e.g., the population of liver-derived cells) with growth media occurs while the liver-derived cells are adhered to a surface (e.g., a surface of a tissue culture dish). In some embodiments, the step of contacting the liver-derived cells (e.g., the population of liver-derived cells) with growth media occurs while the liver-derived cells are in suspension. In some embodiments, the step of contacting the liver-derived cells (e.g., the population of liver-derived cells) with growth media includes two steps, where one step occurs while the liver-derived cells are adhered to a surface (e.g., a surface of a tissue culture dish) and a second step occurring while the liver-derived cells are in suspension.

In some embodiments, the methods provided herein include a step of banking the liver-derived cells. In one embodiment, the liver-derived cells are banked after the step of contacting the liver-derived cells with the growth media.

In some embodiments, cells are seeded in growth media, which can be referred to as "seedtrain" media.

### 4.6.2. Differentiation Media

In some embodiments, a differentiation media comprises base media without any additional additives. Non-limiting examples of base media include: DMEM/F-12, MEM, and IMDM. In some embodiments, a differentiation media comprises base media including serum (e.g., horse serum). For example, differentiation media includes about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19%, or about 20% serum. In some embodiments, differentiation media includes about 2% serum. In some embodiments, the differentiation media is serum free.

In some embodiments, differentiation media includes serum derived from two or more species. In some embodiments, serum is selected from; fetal bovine serum (FBS), chicken serum, and horse serum, or a combination thereof. In some embodiments, differentiation media includes FBS and horse serum, FBS and chicken serum, horse serum and chicken serum, or FBS, chicken serum, and horse serum. In some embodiments, growth media includes about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19% or about 20% of a first serum (e.g., FBS) and about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19% or about 20% of a second serum (e.g., chicken serum).

In some embodiments, differentiation media is selected from: DMEM/F12, about 5% Bovine serum, and about 5% chicken serum; and DMEM/F12, about 2% horse serum, and about 1% ITS (Insulin-Transferrin-Selenium).

In some embodiments, the methods provided herein include contacting a liver-derived cell (e.g., a population of liver-derived cells) with a differentiation media. In such cases, the liver-derived cells (e.g., the population of liver-derived cells) are exposed to the differentiation media for a second period of time. As used herein the phrase "a second period of time" includes any period of time sufficient to allow for differentiation of the liver-derived cells (e.g., the population of liver-derived cells) to non-natively occurring myogenic cells suitable for consumption or any period of time sufficient to allow for dedifferentiation of the liver-derived cells (e.g., the population of liver-derived cells) to a dedifferentiated cell. For example, a second period of time includes from about day 1 to about day 14 (e.g., from about day 1 to about day 13, from about day 1 to about day 12, from about day 1 to about day 11, from about day 1 to about day 10, from about day 1 to about day 9, from about day 1 to about day 8, from about day 1 to about day 7, from about day 1 to about day 6, from about day 1 to about day 5, from about day 1 to about day 4, from about day 2 to about day 14, from about day 2 to about day 13, from about day 2 to about day 12, from about day 2 to about day 11, from about day 2 to about day 10, from about day 2 to about day 9, from about day 2 to about day 8, from about day 2 to about day 7, from about day 2 to about day 6, from about day 2 to about day 5, from about day 2 to about day 4, from about day 3 to about day 14, from about day 3 to about day 13, from about day 3 to about day 12, from about day 3 to about day 11, from about day 3 to about day 10, from about day 3 to about day 9, from about day 3 to about day 8, from about day 3 to about day 7, from about day 3 to about day 6, from about day 3 to about day 5, from about day 3 to about day 4, from about day 4 to about day 14, from about day 4 to about day 13, from about day 4 to about day 12, from about day 4 to about day 11, from about day 4 to about day 10, from about day 4 to about day 9, from about day 4 to about day 8, from about day 4 to about day 7, from about day 4 to about day 6, from about day 4 to about day 5, from about day 5 to about day 14, from about day 5 to about day 14, from about day 5 to about day 13, from about day 5 to about day 12, from about day 5 to about day 11, from about day 5 to about day 10, from about day 5 to about day 9, from about day 5 to about day 8, from about day 5 to about day 7, from about day 5 to about day 6, from about day 6 to about day 14, from about day 6 to about day 13, from about day 6 to about day 12, from about day 6 to about day 11, from about day 6 to about day 10, from about day 6 to about day 9, from about day 6 to about day 8, from about day 6 to about day 7, from about day 7 to about day 13, from about day 7 to about day 12, from about day 7 to about day 11, from about day 7 to about day 10, from about day 8 to about day 13, from about day 8 to about day 12, from about day 8 to about day 10, from about day 9 to about day 13, from about day 9 to about day 12, from about day 9 to about day 11, from about day 10 to about day 13, or from about day 11 to about day 13) of culture, where day 0 is the day the liver-derived cells are first contacted with the growth media.

In some embodiments, a second period of time includes 10, 11, 12, 13, 14, 15, 16, 17, 18, 19, 20, 21, 22, 23, 24, 25, 26, 27, 28, 29 or 30 or more days. In some embodiments, a second period of time includes 1 month, 2 months, 3 months, 4 months, 5 months, 6 months, 7 months, 8 months, 9 months, 10 months, 11 months or 12 months or more.

In some embodiments, a second period of time includes the amount of time needed for the population of liver-derived cells, dedifferentiated cells, or non-natively occurring myogenic cells to have a population doubling level (PDL) (i.e., total number of times the cells in the population have doubled since their primary isolation *in vitro)* of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages.

In some embodiments, the methods provided herein include contacting a liver-derived cell (e.g., a population of liver-derived cells) with two or more different growth differentiation medias.

In some embodiments, the step of contacting the liver-derived cells (e.g., the population of liver-derived cells) with differentiation media occurs while the liver-derived cells are adhered to a surface (e.g., a surface of a tissue culture dish). In some embodiments, the step of contacting the liver-derived cells (e.g., the population of liver-derived cells) with differentiation media occurs while the liver-derived cells are in suspension. In some embodiments, the step of contacting the liver-derived cells (e.g., the population of liver-derived cells) with differentiation media includes two steps, where one step occurs while the liver-derived cells are adhered to a surface (e.g., a surface of a tissue culture dish) and a second step occurring while the liver-derived cells are in suspension. In some embodiments, the step of contacting the liver-derived cells (e.g., the population of liver-derived cells) with differentiation media occurs while the liver-derived cells are adhered to a cultivation infrastructure.

In some embodiments, the methods provided herein include a step of banking the liver-derived cells. In one embodiment, the liver-derived cells are banked after the step of contacting the liver-derived cells with the differentiation media.

In some embodiments, after seeding cells are contacted with a differentiation media, which can be referred to as "seeding" media.

### 4.6.3. Dedifferentiation Media

In some embodiments, the methods provided herein include contacting a liver-derived cell (e.g., a population of liver-derived cells) with a dedifferentiation media.

In some embodiments, a dedifferentiation media comprises base media without any additional additives. Non-limiting examples of base media include: DMEM/F12, MEM, and IMDM. In some embodiments, a dedifferentiation media comprises base media including serum. For example, dedifferentiation media includes about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19%, or about 20% serum. In some embodiments, differentiation media includes about 00% serum. In some embodiments, dedifferentiation media includes about 10% serum. In some embodiments, the growth media is serum free.

In some embodiments, dedifferentiation media includes serum derived from two or more species. In some embodiments, serum is selected from; fetal bovine serum (FBS), chicken serum, and horse serum, or a combination thereof. In some embodiments, dedifferentiation media includes FBS and horse serum, FBS and chicken serum, horse serum and chicken serum, or FBS, chicken serum, and horse serum. In some embodiments, dedifferentiation media includes about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19% or about 20% of a first serum (e.g., FBS) and about 0.1%, about 0.5%, about 1.0%, about 2.0%, about 3.0%, about 4%, about 5%, about 6%, about 7%, about 8%, about 9%, about 10%, about 11%, about 12%, about 13%, about 14%, about 15%, about 16%, about 17%, about 18%, about 19% or about 20% of a second serum (e.g., chicken serum).

In some embodiments, dedifferentiation media includes signaling pathway modulators. In some embodiments, the signaling pathways can either be activated or inhibited. For example, without limitation, Wnt, TGF (Activin A), and BMP signaling pathways can be activated (e.g., using CHIR99021, Activin A, or BMP4, respectively) or inhibited (e.g., using IWR1, A-83-01, or LDN193189, respectively). In some embodiments, dedifferentiation media includes an activator of Wnt signaling (e.g., CHIR99021), an inhibitor of TGF (Activin A) signaling (e.g., A-83-01), and an inhibitor of BMP signaling (e.g., LDN193189).

In some embodiments, the liver-derived cells (e.g., the population of liver-derived cells) are exposed to the dedifferentiation media for an additional period of time. As used herein the phrase "an additional period of time" includes any period of time sufficient to allow for dedifferentiation of a liver-derived cell (e.g., the population of liver-derived cells) to a dedifferentiated cell. For example, an additional period of time includes from about day 1 to about day 14 (e.g., from about day 1 to about day 13, from about day 1 to about day 12, from about day 1 to about day 11, from about day 1 to about day 10, from about day 1 to about day 9, from about day 1 to about day 8, from about day 1 to about day 7, from about day 1 to about day 6, from about day 1 to about day 5, from about day 1 to about day 4, from about day 2 to about day 14, from about day 2 to about day 13, from about day 2 to about day 12, from about day 2 to about day 11, from about day 2 to about day 10, from about day 2 to about day 9, from about day 2 to about day 8, from about day 2 to about day 7, from about day 2 to about day 6, from about day 2 to about day 5, from about day 2 to about day 4, from about day 3 to about day 14, from about day 3 to about day 13, from about day 3 to about day 12, from about day 3 to about day 11, from about day 3 to about day 10, from about day 3 to about day 9, from about day 3 to about day 8, from about day 3 to about day 7, from about day 3 to about day 6, from about day 3 to about day 5, from about day 3 to about day 4, from about day 4 to about day 14, from about day 4 to about day 13, from about day 4 to about day 12, from about day 4 to about day 11, from about day 4 to about day 10, from about day 4 to about day 9, from about day 4 to about day 8, from about day 4 to about day 7, from about day 4 to about day 6, from about day 4 to about day 5, from about day 5 to about day 14, from about day 5 to about day 14, from about day 5 to about day 13, from about day 5 to about day 12, from about day 5 to about day 11, from about day 5 to about day 10, from about day 5 to about day 9, from about day 5 to about day 8, from about day 5 to about day 7, from about day 5 to about day 6, from about day 6 to about day 14, from about day 6 to about day 13, from about day 6 to about day 12, from about day 6 to about day 11, from about day 6 to about day 10, from about day 6 to about day 9, from about day 6 to about day 8, from about day 6 to about day 7, from about day 7 to about day 13, from about day 7 to about day 12, from about day 7 to about day 11, from about day 7 to about day 10, from about day 8 to about day 13, from about day 8 to about day 12, from about day 8 to about day 10, from about day 9 to about day 13, from about day 9 to about day 12, from about day 9 to about day 11, from about day 10 to about day 13, or from about day 11 to about day 13) of culture, where day 0 is the day the liver-derived cells are first contacted with the growth media.

In some embodiments, an additional period of time includes 10, 11, 12, 13, 14, 15, 16, 17, 18, 19, 20, 21, 22, 23, 24, 25, 26, 27, 28, 29 or 30 or more days. In some embodiments, an additional period of time includes 1 month, 2 months, 3 months, 4 months, 5 months, 6 months, 7 months, 8 months, 9 months, 10 months, 11 months or 12 months or more.

In some embodiments, an additional period of time includes the amount of time needed for the population of liver derived cells or dedifferentiated cell to have a population doubling level (PDL) (i.e., total number of times the cells in the population have doubled since their primary isolation *in vitro)* of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages.

In some embodiments, the methods provided herein include contacting a liver-derived cell (e.g., a population of liver-derived cells) with two or more different dedifferentiation medias.

In some embodiments, the step of contacting the liver-derived cells (e.g., the population of liver-derived cells) with dedifferentiation media occurs while the liver-derived cells are adhered to a surface (e.g., a surface of a tissue culture dish). In some embodiments, the step of contacting the liver-derived cells (e.g., the population of liver-derived cells) with dedifferentiation media occurs while the liver-derived cells are in suspension. In some embodiments, the step of contacting the liver-derived cells (e.g., the population of liver-derived cells) with dedifferentiation media includes two steps, where one step occurs while the liver-derived cells are adhered to a surface (e.g., a surface of a tissue culture dish) and a second step occurring while the liver-derived cells are in suspension.

In some embodiments, the methods provided herein include a step of banking the liver-derived cells. In one embodiment, the liver-derived cells are banked after the step of contacting the liver-derived cells with the dedifferentiation media.

### 4.7 Additional Processing of Liver-Derived Cells or Non-Natively Occurring Myogenic Cells

Methods for transdifferentiating and dedifferentiating/differentiating of a population of liver-derived cells provide a population of non-natively occurring myogenic cells suitable for consumption. Once the desired level of transdifferentiation, dedifferentiation, or differentiation (or any combination thereof) is achieved, transdifferentiation, dedifferentiation, or differentiation can be partially or fully terminated and the non-natively occurring myogenic cells can be harvested.

In some embodiments, transdifferentiation, dedifferentiation, and/or differentiation can be partially or fully terminated by partial or complete removal of growth media, dedifferentiation media, and/or differentiation media.

In some embodiments, after partial or complete transdifferentiation, dedifferentiation, and/or differentiation, the population of non-natively occurring myogenic cells can be differentiated (e.g., further differentiation). In some embodiments, the population of liver-derived cells, the population of dedifferentiated cells, or the population of non-natively occurring myogenic cells can be differentiated into a phenotype of interest by contacting the cells with a differentiation agent. For example, if the phenotype of interest for the population of dedifferentiated cells or population of non-natively occurring myogenic cells is skeletal muscle and the population of dedifferentiated cells comprises cells of a non-muscle lineage (e.g., non- myogenic stem cells), the population of dedifferentiated cells or population of non-natively occurring myogenic cells can be contacted with a differentiation agent that would induce the skeletal muscle phenotype into the cells. Exemplary differentiation agents that may induce skeletal muscle phenotype include myogenic transcription factors such as MYOD, MYOG, MYF5, MYF6, PAX3, PAX7, paralogs, orthologs, and genetic variants thereof. Additional non-limiting methods for differentiating cells into a skeletal muscle phenotype are as described in WO/2015/066377, which is herein incorporated by reference in its entirety. In some embodiments, cells of the population of dedifferentiated cells or cells of the population of non-natively occurring myogenic cells can be differentiated into a phenotype of interest without a differentiation agent. For example, if the phenotype of interest for the population of dedifferentiated cells is a skeletal muscle and the population of dedifferentiated cells comprises cells of the skeletal muscle lineage, then these cells may differentiate into the skeletal muscle phenotype on their own without a need for an external differentiation agent.

In some embodiments, the population of dedifferentiated cells or population of non-natively occurring myogenic cells can be processed as a raw, uncooked food product (cultured meat) or as a cooked food product or as a cooked/uncooked food ingredient. In some embodiments, processing comprises withdrawal of the culture medium that supports the viability, survival, growth or expansion (e.g., increase in total protein content of the non-natively occurring myogenic cells) and differentiation of the non-natively occurring myogenic cells. Withdrawal may comprise physical removal of the culture medium or altering the composition of the culture medium, for example, by addition of components that would reduce or prevent further expansion and/or differentiation of the population of dedifferentiated cells or population of non-natively occurring myogenic cells or by depletion of components that support expansion and/or differentiation of the population of dedifferentiated cells or population of non-natively occurring myogenic cells.

### 4.8 Suspension Culture

In some embodiments, the methods provided herein include a step of adapting the population of liver-derived cells for suspension culture. In some embodiments, adapting the population of liver-derived cells for suspension culture is performed in a cultivation infrastructure. In some embodiments, liver-derived cells and/or dedifferentiated cells, when cultured in suspension in a cultivation infrastructure, may adhere to the top, bottom or sidewalls of the cultivation infrastructure. In some embodiments, the population of liver-derived cells, population of dedifferentiated cells, or population of non-natively occurring myogenic cells are cultivated in suspension culture as a single-cell suspension.

### 4.9 Inducing Myogenic-Specific Differentiation.

This disclosure also provides methods for inducing myogenic-specific differentiation of the population of liver-derived cells, dedifferentiated cells, or non-natively occurring myogenic cells. In some embodiments, the method includes adhering the population of liver-derived cells to a cultivation infrastructure and contacting the liver-derived cells with a myogenic differentiation media.

In some embodiments, a myogenic differentiation media comprises base media without any additional additives. Non-limiting examples of base media include: DMEM/F-12, MEM, and IMDM. In some embodiments, a myogenic differentiation media comprises base media including serum. In some embodiments, a myogenic differentiation media comprises ITS (insulin-transferrin-selenium). In some embodiments, a myogenic differentiation media is a serum-free media (e.g., media that is substantially free of serum).

In some embodiments, a myogenic differentiation media comprises base media including bovine serum. For example, myogenic differentiation media includes at least about 0.5%, at least about 1.0%, at least about 2.0%, at least about 3.0%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19%, or at least about 20% bovine serum. In some embodiments, myogenic differentiation media includes at least about 10% bovine serum.

In some embodiments, a myogenic differentiation media comprises base media including chicken serum. For example, myogenic differentiation media includes at least about 0.5% chicken serum, at least about 1.0% chicken serum, at least about 2.0% chicken serum, at least about 3.0% chicken serum, at least about 4% chicken serum, at least about 5% chicken serum, at least about 6% chicken serum, at least about 7% chicken serum, at least about 8% chicken serum, at least about 9% chicken serum, at least about 10% chicken serum, at least about 11% chicken serum, at least about 12% chicken serum, at least about 13% chicken serum, at least about 14% chicken serum, at least about 15% chicken serum, at least about 16% chicken serum, at least about 17% chicken serum, at least about 18% chicken serum, at least about 19% chicken serum or at least about 20% chicken serum. In some embodiments, myogenic differentiation media includes at least about 2% chicken serum.

In some embodiments, a myogenic differentiation media comprises base media including horse serum . For example, myogenic differentiation media includes at least about 0.5%, at least about 1.0%, at least about 2.0%, at least about 3.0%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19, or at least about 20% horse serum. In some embodiments, myogenic differentiation media includes at least about 2% horse serum.

In some embodiments, myogenic differentiation media includes: at least about 10% fetal bovine serum, at least about 5% chicken serum, at least about 2% horse serum, or a combination thereof. In another embodiment, myogenic differentiation media includes at least about 10% bovine serum and at least about 5% chicken serum. In another embodiment, myogenic differentiation media includes at least about 5% bovine serum and at least about 5% chicken serum.

In some embodiments, a population of liver-derived cells, dedifferentiated cells, or non-natively occurring myogenic cells are adhered to cultivation infrastructure and exposed to the myogenic differentiation media for a period of time. In some embodiments, the period of time is any amount of time needed for the population of liver-derived cells, dedifferentiated cells, or non-natively occurring myogenic cells to differentiate to a myogenic cell of interest. Non-limiting examples of a period of time include: about 1 day, about 2 days, about 3 days, about 4 days, about 5 days, about 6 days, about 7 days, about 8 days, about 9 days, about 10 days, about 11 days, about 12 days, about 13 days, about 14 days, or about 15 days, or about 16 days, or about 17 days or about 18 days, or about 19 days, or about 20 days, or about 21 days, or about 22 days, or about 23 days, or about 24 days, or about 25 days, or about 26 days, or about 27 days, or about 28 days, or about 29 days or about 30 or more days.

In some embodiments, the myogenic differentiation media is the same as the differentiation media. In some embodiments, the myogenic differentiation media is different from the differentiation media.

Non-limiting examples of myogenic differentiation are as described in WO2019014652A1, WO2018208628A1, and WO2015066377A1, each of which is herein incorporated by reference in their entireties.

### 4.10 Cultivation Infrastructure

In some embodiments, a cultivation infrastructure may be a tube, a cylinder, a flask, a petri-dish, a multi-well plate, a dish, a vat, a roller bottle, an incubator, a bioreactor, an industrial fermenter and the like.

In some embodiments, a cultivation infrastructure can be of any scale, and support any volume of liver-derived cells and culturing reagents. In some embodiments, the cultivation infrastructure ranges from about 10 µL to about 100,000 L. In some embodiments, the cultivation infrastructure is about 10 µL, about 100 µL, about 1 mL, about 10 mL, about 100 mL, about 1 L, about 10 L, about 100 L, about 1000 L, about 10,000 L, or even about 100,000 L.

In some embodiments, the cultivation infrastructure comprises a substrate. In some embodiments, a cultivation infrastructure may comprise a permeable substrate (e.g. permeable to physiological solutions) or an impermeable substrate (e.g. impermeable to physiological solutions).

In some embodiments, the cultivation infrastructure comprises a primary substrate, which can be a flat, concave, or convex substrate. In some embodiments, the cultivation infrastructure further comprises a secondary substrate, either introduced, or autologous, to direct cellular growth between the substrates, e.g. to direct attachment, proliferation and hypertrophy of cells on a plane perpendicular to the primary substrate.

In some embodiments, the cultivation infrastructure comprises a hydrogel, a liquid cell culture media, or soft agar.

In some embodiments, the cultivation infrastructure does not comprise a substrate to which cells can adhere. In some embodiments, the cultivation infrastructure comprises a suspension culture, e.g. supporting the growth of a self-adhering liver-derived cells, or single-cell suspension of liver-derived cells in a liquid medium.

In some embodiments, the cultivation infrastructure comprises adherent cells (i.e. those cells that adhere to a substrate). In some embodiments, the cultivation infrastructure comprises non-adherent cells (i.e. those cells that do not adhere to a substrate). In some embodiments, the cultivation infrastructure comprises both adherent and non-adherent cells.

### 4.11 Immortalization

Also provided herein are methods for immortalizing (e.g., extending the renewal capacity) a population of liver-derived cells.

In some embodiments, the liver-derived cells undergo spontaneous immortalization.

In some embodiments, immortalization comprises transforming a cell with a telomerase reverse transcriptase (TERT) gene. As used herein, "TERT" refers to telomerase reverse transcriptase (TERT) gene or TERT polypeptide that is a ribonucleoprotein polymerase that maintains telomere ends by addition of the telomere repeat TTAGGG. Telomerase expression plays a role in cellular senescence, as it is normally repressed in postnatal somatic cells resulting in progressive shortening of telomeres. In some embodiments, cells ectopically express the TERT polynucleotide. In some embodiments, the cells are genetically modified and carry stable integrations of one or more copies of the TERT polynucleotide. Exemplary methods for immortalizing a cell line are as described in WO2019014652A1, which is herein incorporated by reference in its entirety.

In some embodiments, increased expression of TERT may be achieved using different approaches. In some embodiments, increased expression of TERT may be achieved by ectopically expressing TERT. In some embodiments, increased expression of TERT may be achieved by introducing targeted mutations in the TERT promoter. In some embodiments, increased expression of TERT may be achieved by activating endogenous TERT expression by an engineered transcriptional activator. In some embodiments, increased expression of TERT may be achieved by transiently transfecting TERT mRNA.

The polynucleotide encoding TERT can be from any organism. The TERT polynucleotide can be from bacteria, plants, fungi, and archaea. The TERT polynucleotide can be from any animal, such as vertebrate and invertebrate animal species. The TERT polynucleotide can be from any vertebrate animal species such as mammals, reptiles, birds, amphibians, and the like. The TERT polynucleotide can be from any mammalian species, such as a human, murine, bovine, porcine, and the like.

In some embodiments, immortalization comprises transforming a cell with a polynucleotide encoding a cyclin- dependent kinase 4 ("CDK4") protein. In some embodiments, immortalization comprises inactivating a gene encoding an inhibitor of cyclin-dependent kinase 4 (CDK4). Exemplary methods for immortalizing a cell line are as described in WO2017124100A1, which is herein incorporated by reference in its entirety.

In some embodiments, immortalization occurs prior to contacting the population of liver-derived cells with a growth media. In some embodiments, immortalization occurs after the liver-derived cells are contacted with growth media. In some embodiments, immortalization occurs after the liver-derived cells are contacted with dedifferentiation media. In some embodiments, immortalization occurs after the liver-derived cells are contacted with differentiation media.

### 4.12 Cells

Also provided herein are any of the liver-derived cells, dedifferentiated cells, or non-natively occurring myogenic cells. In some embodiments, the liver-derived cell is a non-muscle cells. For example, the population of liver-derived cells obtained from the animal (e.g., any of the animals described herein) is substantially free of muscle/myogenic cells.

Also provided herein are any of the liver-derived cells, dedifferentiated cells, or non-natively occurring myogenic cells produced by any of the methods described herein.

Also provided herein are liver-derived cells, dedifferentiated cells, or non-natively occurring myogenic cells that form a self-adherent aggregate (e.g., an aggregate). A self-adherent aggregate refers to masses of viable cells suspended in a physiological liquid medium (e.g., suspension culture) aggregated due to, for example, their (1) adherence to each other (e.g., cadherin cell adhesion) (2) adherence to a basement membrane or other extracellular matrix secreted by the cells (e.g., integrin cell adhesion) or (3) a combination of both. In some embodiments, provided herein are the liver-derived cells, dedifferentiated cells, or non-natively occurring myogenic cells that are cultivated in a suspension culture and form a self-adherent aggregate.

Also provided herein are liver-derived cells (e.g., any of the immortalized cells described herein) including any of the polynucleotides described herein (e.g., polynucleotide encoding a TERT protein).

Also provided herein are aggregates comprising any of the populations of liver-derived cells, any of the dedifferentiated cells, any of the populations of non-natively occurring myogenic cells suitable for consumption, or a combination thereof. In some embodiments, the aggregates are suitable for consumption.

Also provided herein are cell banks comprising any of the populations of liver-derived cells, any of the dedifferentiated cells, any of the populations of non-natively occurring myogenic cells suitable for consumption, or a combination thereof.

### 4.13 Kits and Articles of Manufacture

Also provided herein are kits for transdifferentiating a liver-derived cell into a non-natively occurring myogenic cell. For example, the kits may include a liver-derived cell, growth media (e.g., any of the growth medias described herein), differentiation media (e.g., any of the differentiation medias described herein), and a cultivation infrastructure. Also provided herein are kits for dedifferentiating and differentiating a liver-derived cell to a non-natively occurring myogenic cell. For example, the kits may include a liver-derived cell, growth media (e.g., any of the growth medias described herein), dedifferentiation media (e.g., any of the dedifferentiation medias described herein), differentiation media (e.g., any of the differentiation medias described herein), and a cultivation infrastructure. In such cases, the kit can optionally include a dedifferentiated cell.

Also provided herein are articles of manufacture comprising any one of the compositions, cells, or kits described herein.

### 4.14 Summary of Experimental Observations

Applicant evaluated the ability to make non-natively occurring myogenic cells suitable for consumption using transdifferentiation of a population of liver derived cells. Applicant demonstrated that transdifferentiating a population of liver-derived cells to a population of non-natively occurring myogenic cells results in the population of non-natively occurring myogenic cell having robust expression of muscle-specific expression markers (e.g., MyHC) and ability to form myotubes, as well as having increased total protein weight as compared to controls.

Overall, this work demonstrated that liver-derived cells can serve as a source of a myogenic cell line suitable for consumption. In particular, the transdifferentiated cells were spontaneously immortalized and adapted to suspension and adherent culture without losing the ability to differentiate into myogenic cells. The ability to maintain, and even improve on the differentiation capacity (i.e., the ability to form non-natively occurring myogenic cells) is critical given that cells typically lose differentiation capacity following immortalization, adaptation to suspension, and extended periods in culture. Therefore, this disclosure is powerful as it provides methods for producing non-natively occurring myogenic cells suitable for consumption without the need for genetic engineering.

### 5 EXAMPLES

### 5.7 Experimental Procedures/Methods

### 5.7.4 Isolation of liver-derived cells, adaptation to suspension culture, and adherence/culture in a cultivation infrastructure

**Phase I:** Isolation of a population of liver derived cells.

In a non-limiting example, liver cells were procured from chicken embryonic day 16 liver using the following steps. A chicken carcass was rinsed in a beaker with 50 mL of 70% (vol/vol) ethanol for 2 minutes. The liver was aseptically removed and immediately transferred into ice-cold phosphate-buffered saline (PBS; Ca2⁺ and Mg2⁺ free and 5% (vol/vol) penicillin/streptomycin) for 2-3 minutes. The liver was rinsed again in PBS - (calcium free and magnesium free) buffer. The livers were then minced with a sterile scalpel blade into pieces less than or equal to 1 mm³ in 2 mL of 0.05% trypsin. The pieces were further processed by breaking apart the tissue pieces (i.e., the less than or equal to 1 mm³) using glass slides.

The tissue pieces were transferred into a sterile conical tube. Tissue pieces were further digested by incubating with 0.25% (wt./vol.) trypsin having 0.02% (wt./vol.) EDTA (~1 mL of 0.25% trypsin for every 100 mg of tissue). The tissue pieces and trypsin/EDTA were mixed using intermittent pipetting along with stirring at 37°C in a water bath for 4 minutes. The cell suspension was allowed to stand for 1 minute. The supernatant containing single cell suspension was collected into a fresh conical tube with the tubes containing the single cell suspension kept on ice.

ME18 media was added to the single cell suspension and the digestion step was repeated three times. The cell suspension from each digestion was pooled and centrifuged at 3,500 rpm for 10 minutes at 4°C. The cell pellet was suspended in ME58 media and seeded into a 25 cm² plastic culture flask (scale as appropriate) in the presence of 6 mL of growth medium ME58 (e.g., bovine serum, chicken serum, and FGF2) with 5% anti-anti. The cells were plated on precoated culture flasks with 1% gelatin and incubated in 95% air and 5% CO₂ at 37°C for ~2-3 hours to allow differential attachment of fibroblast cells. The non-adherent cells (i.e., liver-derived cells) were transferred to a sterile tube and transferred to a culture flask. Media was changed every 3 days. Cells were maintained in culture and passaged with a split ratio of 1:3, thereby establishing a cell line of liver-derived cells. The adherent cells were harvested once the cells reached confluency by removing the medium and adding 2 mL of 0.25% (wt./vol.) trypsin having 0.02% EDTA (wt./vol.).

To expand the line of liver-derived cells, the liver-derived cell cultures were incubated in growth media ME58 (e.g., bovine serum, chicken serum, and FGF2). Cell growth was characterized by measuring population doubling level (PDL). Cells exhibited robust doubling time, for example, the liver-derived cells had a population doubling time of about 24 hours or less.

Liver-derived cells were banked at various time points during the protocol, including when the liver-derived cells were established as a cell line and also banked again following expansion. These cell banks provide a population of liver-derived cells that can serve as a renewable source of non-natively occurring myogenic cells.

In addition to assessing growth, the liver-derived cells were tested for differentiation of capacity by contacting the liver-derived cells with differentiation media (i.e., DMEM/F12 with 2% horse serum). In particular, the liver-derived cells were placed into a combination of starvation and differentiation media. Without wishing to be bound by theory, the liver-derived cells were contacted with the starvation/differentiation media combination to force the cells to use energy to differentiate rather than for growth.

The following assays were used to determine the ability of the liver-derived cells to differentiate to non-natively occurring myogenic cells and determine total protein content of the differentiated cells. First, Cells were stained for myogenic markers including MyHC and assessed for ability to form myotubes, based in part on the MyHC staining. Next, BCA assays (ThermoFisher, Cat No. 23227) were used to determine total protein content. Finally, total protein content was measured following drying to determine how much moisture the tissue (e.g., non-liver derived cells) contained. For this measurement, BCA was used to determine dry weight protein percentage.

In a production run, cultures will be monitored to ensure tissue (e.g., liver-derived cells) is thick enough (e.g. enough cell layers have formed), before switching to differentiation media.

**Phase II:** Liver-derived cells were adapted for suspension, and optionally, spontaneously immortalized.

In this phase, prior to differentiation liver-derived cell lines were adapted for suspension and/or immortalized (e.g., according to the methods provided herein). Non-limiting examples of adapting cells for suspension culture are as described in WO2018208628A1, which is herein incorporated by reference in its entirety.

**Phase III:** Myogenic differentiation and cultivation infrastructure.

In this phase, liver-derived cells in suspension are reattached to a solid surface (plastic/stainless steel of a cultivation infrastructure) and contacted with differentiation media (e.g., any of the differentiation medias described herein). Alternatively, the liver-derived cells have already been contacted with differentiation media while in suspension and prior to reattaching to the solid surface (plastic/stainless steel of a cultivation infrastructure. Following either approach, the differentiated cells are then assayed for myotube formation and expression of meat proteins.

Non-limiting examples of adhering cells to a cultivation infrastructure are as described in WO2018208628A1, which is herein incorporated by reference in its entirety. The resulting population of non-natively occurring myogenic cells can be processed as a raw, uncooked food product (cultured meat) or as a cooked food product or as a cooked/uncooked food ingredient.

### 5.7.5 Assessment of Myogenicity

Using immunohistochemistry. Cells were fixed with 4% paraformaldehyde (PFA) and washed. Cells were permeabilized with 0.05% PBS-T (triton-x), blocked with normal goat serum (Millipore Sigma), and were incubated with antibodies (ThermoFisher) specific for MyHC, a muscle cell marker. Nuclei were stained with DAPI (4',6-diamidino-2-phenylindole) (ThermoFisher) to visualize and represent all cells in the imaged area. All immunostained samples were imaged by Cytation 5 (Biotek) microscope and analyzed using Gen5 software.

Alternatively, myogenicity is assessed using qRT-PCR (real-time quantitative reverse transcription). Messenger RNA (mRNA) is isolated from cells to examine gene expression with probes specifically designed to amplify target genes to characterize cell lines. Identical quantity of mRNA is reverse transcribed to generate cDNA. Each cDNA is submitted to quantitative PCR (qPCR) to assess the expression of myogenic factors relative to a housekeeping gene. Expression of higher levels of Myf6, MyoD, MyoG, MYMK, MyHC suggest higher myogenic potential to differentiate. Additionally, high levels of MyHC1e are indicative of cells that can mature to form myotubes.

### 5.8 Example 1: TERT-immortalized, suspension adapted myoblasts lose their differentiation capacity.

This experiment was designed to test the hypothesis that TERT-immortalized, suspension adapted myoblasts experience reduced differentiation capacity over time in culture.

The results of these experiments are shown in **FIGs. 2A-2D**. **FIGs. 2A-2D** compare MyHC expression and myotube formation (e.g., MyHC staining with myotubes indicated as elongated tendrils) in TERT-immortalized chicken myoblasts (**FIGs. 2A-2B**) and primary myoblasts (**FIGs. 2C-2D**). TERT-immortalized myoblasts were generated by transforming myoblasts with a nucleic acid encoding telomerase reverse transcriptase (TERT), the catalytic subunit of the telomerase. **FIG. 2B** shows no MyHC expression and no myotube formation in the TERT-immortalized chicken myoblasts, thereby demonstrating that TERT-immortalized chicken myoblasts lose their myogenic potential in culture. **FIG. 2D** shows primary chicken myoblasts include myotubes expressing myosin heavy chain (MyHC) (green elongated cells).

This data suggested that TERT-immortalized myoblasts experience reduced differentiation capacity following extended culture periods, and therefore, highlighted a need for finding cells capable of expansion without losing their ability to differentiate into myogenic cells suitable for consumption.

### 5.9 Example 2: Development of myogenic suspension cells from non-skeletal muscle derived cells

Liver-derived Inky/Kawhi lines were assessed for their myogenic differentiation potential following culturing according to the methods described herein.

For these experiments, chicken fibroblast cells (i.e., 1312 chicken fibroblasts) grown in suspension were seeded in a roller bottle. Following seeding by the chicken fibroblasts, rodentia liver cells were added to the roller bottles and co-cultured with the chicken fibroblasts. Alternatively, conditioned media from a chicken fibroblast cell culture was added to the roller flasks containing the rodentia liver cells. In this case, media from a chicken fibroblast cell culture was collected, centrifuged, and the supernatant was removed and added to the roller flasks.

The results are presented in **FIGs. 4A-4C**. The rodentia liver-derived Inky/Kawhi cells adapted to suspension culture as shown by the representative image of **FIG. 4A**. In addition, the rodentia-liver derived Inky/Kawhi cells underwent spontaneous immortalization as indicated by the rebounding of the viable cell counts following each passage (i.e., each trough indicates a passage where cell density was adjusted based on color change of phenol red indicator in culture medium) as shown in **FIG. 4B**. Finally, after switching to differentiation media and adhering the liver-derived cells to a cultivation infrastructure, the liver-derived Inky/Kawhi cells showed robust expression of MyHC at days 7, day 10, and day 13 following adherence (see **FIG. 4C**). Taken together, this data suggested that liver-derived cells can be adapted for suspension culture, undergo spontaneous immortalization, and differentiate into and maintain MyHC expression during culture. These experiments establish that liver-derived cells can be used as a source of myogenic cells.

### 5.10 Example 3: Liver-derived cells form myosin heavy chain (MyHC) positive myotubes

This experiment was performed to assess the differentiation potential of chicken embryonic day 16 liver cells. In particular, the chicken embryonic liver cells were assessed for their ability to form MyHC⁺ myotubes.

For this experiment, following isolation from the liver, the liver-derived cells were cultured for either five (i.e., P5) or eight (i.e., P8) passages prior to being plated at day 0. On day 0, liver-derived cells were plated in 96-well black plates precoated with collagen/fibronectin at 5000 cells/well in proliferation media including 100µl ME58 and 100µl 2X M9. Positive control chicken myoblasts immortalized with TERT (e.g., 8D-TERT cells) and negative control chicken skin fibroblasts (e.g., 1312 cells) were also plated at 5000 cells/well in the same proliferation media (i.e., 100µl ME58 and 100µl 2X M9). On day 3, proliferation media was replaced with differentiation media including 2% horse serum in DMEM/F12. Liver-derived cells, positive controls and negative controls were assessed at day 7 for MyHC protein expression using immunofluorescence.

**FIGs. 5A-L** shows representative images of MyHC staining at day 7. Myotubes are indicated as (light green) elongated tendrils. Cells were imaged via florescence microscopy at 10x magnification power. Cells were counterstained with DAPI (blue signal) for nuclei and with myosin heavy chain antibody conjugated with Alexa488 (green signal). As shown in **FIGs. 5A-L**, chicken embryonic liver-derived cells express MyHC protein (P8: **FIGs. 5A-5C** and P5: **FIGs. 5D-5F**) while chicken skin fibroblasts (**FIG. 5J-5L**) had few MyHC⁺ cells. As shown in **FIGs. 2A-2D**, immortalized chicken myoblasts (**FIG. 5G-5I**) had few cells expressing MyHC, which indicates that overexpression of TERT limited the ability of these cells to differentiate into myotubes. This further highlights the unpredictability and the importance of this work by showing that overexpression of TERT in some cell types may limit myogenic differentiation potential. Overall, this data suggested that cells derived from chicken livers at embryonic day 16 harbor myogenic potential.

### 5.11 Example 4: Liver-derived cells form myosin heavy chain (MyHC) positive myotubes under starvation conditions

Chicken embryonic day 16 liver cells were assessed for their potential to form MyHC⁺ myotubes during media starvation.

For this experiment, following isolation from the liver, the liver-derived cells were cultured for either five (i.e., P5) or eight (i.e., P8) passages prior to being plated at day 0. On day 0, liver-derived cells were plated in 96-well black plates precoated with collagen/fibronectin at 5000 cells/well in proliferation media including 100µl ME58 and 100µl 2X M9. Positive control chicken myoblasts immortalized with TERT (e.g., 8D-TERT cells) and negative control chicken skin fibroblasts (e.g., 1312 cells) were also plated at 5000 cells/well in the same proliferation media (i.e., 100µl ME58 and 100µl 2X M9). In contrast to the experiment described in Example 3, proliferation media was not replaced at day 3. Liver-derived cells, positive controls, and negative controls were assessed at day 7 for MyHC protein expression using immunofluorescence.

**FIGs. 6A-L** shows representative images of MyHC staining at day 7. Myotubes are indicated as (light green) elongated tendrils. Cells were imaged via florescence microscope at 10x magnification power. Cells were counterstained with DAPI (blue signal) for nuclei and with myosin heavy chain antibody conjugated with Alexa488 (green signal). As shown in **FIGs. 6A-L****,** chicken embryonic liver-derived cells express MyHC protein (P8: **FIGs. 6A-6C** and P5: **FIGs. 6D-6F**), while chicken skin fibroblasts (**FIG. 6J-****6L**) had little or no expression of MyHC protein. Similar to example 4 (**FIGs. 5G-5I**), immortalized chicken myoblasts (**FIG. 6G-6I**) had few MyHC⁺ cells, which indicates that overexpression of TERT limited the ability of these cells to differentiate into myotubes. This again highlights the unpredictability and the importance of this work by showing that overexpression of TERT in some cell types may limit myogenic differentiation potential. Taken together, this data suggested that cells derived from chicken livers at embryonic day 16 harbor myogenic potential even under starvation conditions and when not exposed to differentiation media.

### 5.12 Example 5: Time course shows myotube formation starting at day 1 of culture with differentiation media

A time course of MyHC staining and myotube formation was performed on the chicken embryonic day 16 liver cells to assess when myogenic markers were first expressed.

For this experiment, the experimental setup was the same as described in Example 3 except for the following changes. 8D primary myoblasts were used as positive control rather than chicken myoblasts immortalized with TERT. Starting at day 3, when proliferation media was replaced with differentiation media (DMEM/F12 + 2% horse serum), each condition (i.e., P5, P8, 8D-TERT, and 1312 cells) was stained for MyHC and DAPI. Each condition was also assessed for MyHC and DAPI staining on each of day 4, day 5, and day 7.

**FIGs. 7A-L** shows representative images of MyHC staining for each condition at days 3, 4, 5, and 7. Myotubes are indicated as (light green) elongated tendrils. Cells were imaged via florescence microscope at 10x magnification power. Cells were counterstained with DAPI (blue signal) for nuclei and with myosin heavy chain antibody conjugated with Alexa488 (green signal). As shown in **FIGs. 7A-L**, chicken liver-derived cells (**FIG. 7B**) and 8D primary myoblasts (**FIG. 7F**) express MyHC protein as early as 1 day following the switch to differentiation media. In contrast, chicken skin fibroblasts (**FIG. 7J**) were negative for MyHC at each time point measured. This data suggested that cells derived from chicken livers at embryonic day 16 harbor robust myogenic differentiation potential that can be exploited shortly after exposure to conditions sufficient to induce myogenic differentiation.

### 5.13 Example 6: Liver-derived cells demonstrate myogenic differential potential under various media conditions

Chicken embryonic day 16 liver cells were assessed for their myogenic differentiation potential in various media conditions.

On day 0, liver-derived cells were plated in non-coated 96-well plates at 5000 cells/well in 200µl of the specified seedtrain media. Negative control chicken skin fibroblasts (e.g., 1312 cells) and positive control 8D primary chicken myoblasts (e.g., 8D cells) were also plated at 5000 cells/well in the seedtrain media. Seedtrain media included a media selected from: ME58: DMEM/F12, about 20% FBS, and about 5% chicken serum; ME9: DMEM/F12, about 20% FBS, about 5% chicken serum, CHIR99021, A-83-01, and LDN193189; and ME93: DMEM/F12, about 10% Bovine serum, and about 5% chicken serum.

Once the cells reach 90-100% confluence on day 3, seedtrain media was replaced with 200µl of the specified seeding media. Seeding media included a media selected from: ME94: DMEM/F12, about 5% Bovine serum, and about 5% Chicken serum; "Starvation:" the growth/seed train media was not changed or replenished (i.e., nutrient depletion); and "2% HS:" DMEM/F12, about 2% Horse serum, and about 1% ITS (Insulin-Transferrin-Selenium).

Liver-derived cells, positive controls and negative controls were assessed at day 7 for MyHC protein expression using immunofluorescence.

**FIGs. 8A-8O** shows representative images of MyHC staining for each combination of seedtrain and seeding media (as noted at the top of each column in **FIGs. 8A-8O**). Myotubes are indicated as (light green) elongated tendrils. Cells were imaged via florescence microscope at 10x magnification power. Cells were counterstained with DAPI (blue signal) for nuclei and with myosin heavy chain antibody conjugated with Alexa488 (green signal). As shown in **FIGs. 8A-8O**, chicken liver-derived cells (**FIGs. 8K-8O****)** and 8D primary myoblasts both express (**FIGs. 8F-8J**) MyHC protein in each combination of seedtrain and seeding media except the chicken liver-derived cells did not express MyHC in the "M9 / starvation" combination. In contrast, the negative control cells did not express MyHC in any combination of seedtrain and seeding media.

This data suggested that transdifferentiation of chicken liver-derived cells to myogenic cells is robust in that it occurs in various combinations of seedtrain and seeding media.

### 5.14 Example 7: Chicken liver-derived cells display similar morphology to positive controls when cultured in roller bottles

Following adaptation to and expansion in suspension culture, the chicken embryonic day 16 liver derived cells were adhered to a roller bottle, which is an exemplary cultivation infrastructure.

Cells grown in suspension culture are seeded in roller bottles in media that promotes adherent cell growth. Following adherence, cell culture media is switched to growth media (e.g., any of the growth medias described herein) and cells proliferate. Following proliferation, cell culture media is switched to differentiation media (e.g., any of the differentiation medias described herein).

**FIGs. 9A-B** shows representative bright field images of chicken skin fibroblasts (e.g., 1312 cells) (**FIG. 9A**) and chicken embryonic liver-derived cells (**FIG. 9B**) cultured in roller bottles. Cells were imaged at 4x magnification power. Chicken embryo liver-derived cells (**FIG. 9B**) demonstrated cell alignment and swirl morphology on a plastic surface-consistent with myoblast morphology (not shown). This data suggest chicken liver-derived cells can be adapted to suspension culture, expanded, and adhered to a cultivation infrastructure, thereby showing that this method is scalable and capable of producing non-natively occurring myogenic cells suitable for consumption.

### 5.15 Example 8: Chicken liver-derived cells transdifferentiated to myogenic cells have higher total protein than compared to chicken fibroblasts

The non-natively occurring myogenic cells produced as a result of the transdifferentiation of the chicken liver-derived cells were characterized based on total protein. The myogenic cells were compared to positive and negative controls as well as averages for chicken fibroblasts grown in adherent conditions.

Total protein was harvested according to standard methods known in the art and calculated either as wet weight (w/w% BCA) or dry weight (Protein % solids). The results are shown in FIGs. 10 and 11.

As shown in **FIG. 10**, non-natively occurring myogenic cells transdifferentiated from chicken embryonic liver cells that were cultured in growth media or differentiation media (e.g., DMEM/F12 + 2% horse serum) both produced statistically significant differences in total protein weight (w/w% BCA) as compared to total protein weight for the chicken fibroblasts grown in adherent conditions. The chicken skin fibroblasts (e.g., 1312 cells) did not produce statistically significant differences in total protein weight (w/w% BCA) as compared to the chicken fibroblasts grown in adherent conditions. Statistically significant differences between the means were determined by an ANOVA one-way test with P-values indicated with asterisks. N=4. ^{∗∗∗} p <0.001; ^{∗∗∗∗} p < 0.0001, ns = not significant.

As shown in **FIG. 11b,** non-natively occurring myogenic cells transdifferentiated from chicken embryonic liver cells that were cultured in standard media or differentiation media both produced statistically significant difference in total protein weight (w/w% BCA) as compared to total protein weight for chicken fibroblasts grown in adherent conditions. In addition, the chicken skin fibroblasts (e.g., 1312 cells) also produce statistically significant differences in total protein weight (w/w% BCA) as compared to the average for chicken fibroblasts grown in adherent conditions. Statistically significant differences between the means were determined by an ANOVA one-way test with P-values indicated with asterisks. N=4. ^{∗∗∗} p <0.001; ^{∗∗∗∗} p < 0.0001, ns = not significant.

Overall, this data showed that non-natively occurring myogenic cells transdifferentiated from chicken embryonic liver cells provide an improved alternative to current methods, at least in terms of total protein content.

### 6 EQUIVALENTS AND INCORPORATION BY REFERENCE

All references cited herein are incorporated by reference to the same extent as if each individual publication, database entry (e.g., Genbank sequences or GeneID entries), patent application, or patent, was specifically and individually indicated incorporated by reference in its entirety, for all purposes. This statement of incorporation by reference is intended by Applicants, pursuant to 37 C.F.R. §1.57(b)(1), to relate to each and every individual publication, database entry (e.g., Genbank sequences or GeneID entries), patent application, or patent, each of which is clearly identified in compliance with 37 C.F.R. §1.57(b)(2), even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

While the invention has been particularly shown and described with reference to a preferred embodiment and various alternate embodiments, it is understood by persons skilled in the relevant art that various changes in form and details can be made therein without departing from the spirit and scope of the invention.

### 7. ADDITIONAL STATEMENTS OF INVENTION

The following numbered clauses, describing aspects of the invention, are part of the description.
1. A method for transdifferentiating a liver-derived cell to a non-natively occurring myogenic cell suitable for consumption, the method comprising:
   (a) contacting a population of liver-derived cells with a growth media; and
   (b) inducing transdifferentiation of one or more liver-derived cell into one or more non-natively occurring myogenic cell suitable for consumption by contacting the population of liver-derived cells with a differentiation media,
      wherein the non-natively myogenic cell comprises greater total protein (w/w%) as compared to cells not exposed to the growth media, the differentiation media, or a combination thereof.
2. The method of clause 1, further comprising prior to step (a):
   obtaining a population of liver-derived cells from a liver of an animal, wherein the animal is selected from a livestock, a poultry, a game, or an aquatic species.
3. The method of clause 1 or 2, wherein the liver of an animal is an embryonic liver, a fetal liver, or a neo-natal liver.
4. The method of clause 2 or 3, wherein the age of the animal is about embryonic day 16 (E16).
5. The method of any one of clauses 1-4, wherein step (a) further comprises adhering the population of liver-derived cells to a substrate.
6. The method of clause 5, wherein the population of liver-derived cells in step (a) comprises a subpopulation adhered to the substrate, a subpopulation that is non-adherent, or both.
7. The method of any one of clauses 1-6, further comprising:
   adapting the population of liver-derived cells for suspension culture.
8. The method of any one of clauses 1-7, wherein the contacting of the population of liver derived cells with the growth media results in an increase in the cell proliferation rate of the population of liver-derived cells.
9. The method of clause 8, wherein the increase in cell proliferation rate enables a population doubling level (PDL) (i.e., total number of times the cells in the population have doubled since their primary isolation *in vitro*) of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the population of liver-derived cells.
10. The method of any one of clauses 1-9, wherein inducing transdifferentiation of a liver-derived cell by contacting the population of liver-derived cells with a differentiation media results in an increase in the cell proliferation rate of the population of liver-derived cells.
11. The method of clause 10, wherein the increase in cell proliferation rate enables a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the population of liver-derived cells.
12. The method of any one of clauses 1-11, wherein the method further comprises:
   an immortalizing step, wherein the population of liver-derived cells, the non-natively occurring myogenic cells, or both are immortalized.
13. The method of clause 12, wherein the immortalization comprises spontaneous immortalization as a result of contacting the population of liver-derived cells, the non-natively occurring myogenic cells, or both with the growth media.
14. The method of clause 13, wherein the immortalization is selected from a method comprising: transducing with a polynucleotide encoding TERT, transducing with a polynucleotide encoding CDK4/6, transducing with a polynucleotide Cyclin D1, inactivating a gene encoding an inhibitor of cyclin-dependent kinase 4/6 (CDK4/6), inactivating a gene encoding an inhibitor of Cyclin D1, or a combination thereof.
15. The method of any one of clauses 1-14, further comprising:
   adhering the population of liver-derived cells, non-natively occurring myogenic cells, or both to a cultivation infrastructure.
16. The method of clause 15, wherein the adhering step comprises adhering the cells to cultivation infrastructure in the presence of differentiation medium.
17. The method of any one of clauses 1-16, wherein the non-natively occurring myogenic cell suitable for consumption comprises at least 1.5-fold greater total protein as compared to (i) a myogenic cell not exposed to any of the methods of clauses 1-16 or (ii) chicken fibroblasts grown in adherent conditions.
18. The method of any one of clauses 1-16, wherein the population of non-natively occurring myogenic cells suitable for consumption have higher proliferation rates as compared to (i) a myogenic cell not exposed to any of the methods of clauses 1-16 or (ii) chicken fibroblasts grown in adherent conditions.
19. The method of any one of clauses 1-16, wherein the population non-natively occurring myogenic cells exhibits increased Myogenin expression, increases MyHC expression, and increased myotube formation, or a combination thereof, as compared to (i) a myogenic cell not exposed to any of the methods of clauses 1-16 or (ii) chicken fibroblasts grown in adherent conditions.
20. The method of any one of clauses 1-19, wherein the growth media is selected from:
   (i)DMEM/F12, about 20% FBS, and about 5% chicken serum;
   (ii) DMEM/F12, about 20% FBS, about 5% chicken serum, CHIR99021, A-83-01, and LDN193189; and
   (iii) DMEM/F12, about 10% FBS, and about 5% chicken serum.
21. The method of any one of clauses 1-20, wherein the differentiation media is selected from:
   (i) DMEM/F12, about 5% FBS, and about 5% Chicken serum; and
   (ii) DMEM/F12, about 2% Horse serum, and about 1% ITS (Insulin-Transferrin-Selenium).
22. The method of any one of clauses 1-21, wherein the population of liver-derived cells is substantially free of cells that have acquired mutations that increase cell proliferation rates.
23. The method of any one of clauses 1-22, wherein the population of liver-derived cells is substantially free of multipotent stem cells and mesenchymal stem cells.
24. The method of any one of clauses 1-23, wherein the population of liver-derived cells is substantially free of hematopoietic cells.
25. The method of any one of clauses 1-24, wherein the population of liver-derived cells are obtained from *Gallus gallus*, *Bos taurus*, *Sous scrofa*, *Meleagris gallopavo*, *Anas platyrynchos, Salmo salar, Thunnus thynnus, Ovis aries, Coturnix coturnix, Capra aegagrus hircus*, or *Homarus americanus.*
26. A non-natively occurring myogenic cell suitable for consumption produced using the method of any one of clauses 1-25.
27. An aggregate comprising a population of non-natively occurring myogenic cells suitable for consumption produced using the methods of any one of clauses 1-25.
28. A cell bank comprising the population of non-natively occurring myogenic cells produced using the methods of any one of clauses 1-25.
29. A method of making a non-natively occurring myogenic cell suitable for consumption, the method comprising:
   (a) contacting a population of liver-derived cells with a growth media;
   (b) inducing dedifferentiation of a liver-derived cell into a dedifferentiated cell by contacting the population liver derived cells with a dedifferentiation media;
   (c) inducing differentiation of the dedifferentiated cell into a non-natively occurring myogenic cell suitable for consumption by contacting the dedifferentiated cells with a differentiation media,
      wherein the non-natively myogenic cell comprises greater total protein (w/w%) as compared to cells not exposed to the growth media, the suspension culture, the differentiation media, or a combination thereof.
30. The method of clause 29, further comprising prior to step (a):
   obtaining a population of liver-derived cells from a liver of an animal, wherein the animal is selected from a livestock, a poultry, a game, or an aquatic species.
31. The method of clause 30, wherein the liver of an animal is an embryonic liver, a fetal liver, or a neo-natal liver.
32. The method of clause 30, wherein the age of the animal is about embryonic day 16 (E16).
33. The method of any one of clauses 29-32, wherein step (a) further comprises adhering the population of liver-derived cells to a substrate.
34. The method of any one of clauses 29-33, wherein the population of liver-derived cells in step (b) comprises a subpopulation adhered to the substrate, a subpopulation that is non-adherent, or both.
35. The method of clause 29, further comprising:
   adapting the population of liver-derived cells for suspension culture..
36. The method of any one of clauses 29-35, wherein the contacting of the population of liver derived cells with the growth media results in an increase in the cell proliferation rate of the population of liver-derived cells.
37. The method of clause 36, wherein the increase in cell proliferation rate enables a population doubling level (PDL) (i.e., total number of times the cells in the population have doubled since their primary isolation *in vitro*) of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the population of liver-derived cells.
38. The method of any one of clauses 29-37, wherein inducing dedifferentiation of the population of liver derived cells with the dedifferentiation media results in an increase in the cell proliferation rate of the population of liver-derived cells.
39. The method of clause 38, wherein increase in cell proliferation rate enables a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the population of liver-derived cells.
40. The method of any one of clauses 29-39, wherein inducing differentiation of the dedifferentiated cell into a non-natively occurring myogenic cells suitable for consumption by contacting the dedifferentiated cells with the differentiation media results in an increase in the cell proliferation rate of the dedifferentiated cells.
41. The method of clause 40, wherein increase in cell proliferation rate enables a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the dedifferentiated cells.
42. The method of any one of clauses 29-41, wherein the method further comprises:
   an immortalizing step, wherein the population of liver-derived cells, the dedifferentiated cells, the non-natively occurring myogenic cells, or a combination thereof, are immortalized.
43. The method of clause 42, wherein the immortalization comprises spontaneous immortalization as a result of contacting the population of liver-derived cells, the dedifferentiated cells, the non-natively occurring myogenic cells, or a combination thereof, with the growth media.
44. The method of clause 42, wherein the immortalization is selected from a method comprising: transducing with a polynucleotide encoding TERT, transducing with a polynucleotide encoding CDK4/6, transducing with a polynucleotide Cyclin D1, inactivating a gene encoding an inhibitor of cyclin-dependent kinase 4/6 (CDK4/6), inactivating a gene encoding an inhibitor of Cyclin D1, or a combination thereof.
45. The method of any one of clauses 29-44, further comprising:
   adhering the population of liver-derived cells, non-natively occurring myogenic cells, or both to a cultivation infrastructure.
46. The method of any one of clause 29-45, wherein the adhering step comprises adhering the cells to cultivation infrastructure in the presence of differentiation medium.
47. The method of any one of clauses 29-46, wherein the non-natively occurring myogenic cell comprises at least 1.5-fold greater total protein as compared to (i) a myogenic cell not exposed to the method clause 1 or (ii) chicken fibroblasts grown in adherent conditions.
48. The method of any one of clauses 29-47, wherein the population of non-natively occurring myogenic cell suitable for consumption have higher proliferation rates as compared to (i) a myogenic cell not exposed to the method of clause 1 or (ii) chicken fibroblasts grown in adherent conditions.
49. The method of any one of clauses 29-48, wherein the population non-natively occurring myogenic cell exhibits increased Myogenin expression, increases MyHC expression, and increased myotube formation, or a combination thereof, as compared to (i) a myogenic cell not exposed to the method of clause 1 or (ii) chicken fibroblasts grown in adherent conditions.
50. The method of any one of clauses 29-49, wherein the growth media is selected from:
   (i)DMEM/F12, about 20% FBS, and about 5% chicken serum;
   (ii) DMEM/F12, about 20% FBS, about 5% chicken serum, CHIR99021, A-83-01, and LDN193189; and
   (iii) DMEM/F12, about 10% FBS, and about 5% chicken serum.
51. The method of any one of clauses 29-50, wherein the differentiation media is selected from:
   (i) DMEM/F12, about 5% FBS, and about 5% Chicken serum; and
   (ii) DMEM/F12, about 2% Horse serum, and about 1% ITS (Insulin-Transferrin-Selenium).
52. The method of any one of clauses 29-51, wherein the dedifferentiation media is selected from:
   (i) DMEM/F12, about 20% FBS, and about 5% chicken serum;
   (ii) DMEM/F12, about 20% FBS, about 5% chicken serum, CHIR99021, A-83-01, and LDN193189; and
   (iii) DMEM/F12, about 10% FBS, and about 5% chicken serum.
53. The method of any one of clauses 29-52, wherein the differentiation media and dedifferentiation media are the same.
54. The method of any one of clauses 29-53, wherein the differentiation media and dedifferentiation media are different.
55. The method of any one of clauses 29-54, wherein the dedifferentiated cell is a liver progenitor-like cell.
56. The method of any of clauses 29-55, wherein the dedifferentiated cells expresses one or more markers selected from: Sox9, Foxl1, Lgr5, and Pkh2.
57. The method of any one of clauses 29-56, wherein following step (c) the population of liver-derived cells is substantially free of cells expressing one or more liver markers.
58. The method of any one of clauses 29-57, further comprising a step of purifying the dedifferentiated cells.
59. The method of any one of clauses 1-58, wherein the population of liver-derived cells is substantially free of cells that have acquired mutations that increase cell proliferation rates.
60. The method of any one of clauses 1-59, wherein the population of liver-derived cells is substantially free of multipotent stem cells and mesenchymal stem cells.
61. The method of any one of clauses 1-60, wherein the population of liver-derived cells is substantially free of hematopoietic cells.
62. The method of any one of clauses 1-61, wherein the population of liver-derived cells are obtained from *Gallus gallus*, *Bos taurus*, *Sous scrofa*, *Meleagris gallopavo*, *Anas platyrynchos*, *Salmo salar, Thunnus thynnus, Ovis aries, Coturnix coturnix, Capra aegagrus hircus*, or *Homarus americanus.*
63. A non-natively occurring myogenic cell suitable for consumption produced using the method of any one of clauses 29-62.
64. An aggregate comprising a population of non-natively occurring myogenic cells produced using the methods of any one of clauses 29-63 suitable for consumption.
65. A cell bank comprising the population of non-natively occurring myogenic cells produced using the methods of any one of clauses 29-64.

## Claims

1. A method of making one or more non-natively occurring myogenic cells suitable for consumption, the method comprising:
(a) contacting a population of liver-derived cells with a growth media, wherein the growth media comprises a modulator of Activin-A mediated signaling, a modulator of BMP-mediated signaling, a modulator of Wnt-mediated signaling, or a combination thereof;
(b) adapting the population of liver-derived cells for suspension culture; and
(c) inducing transdifferentiation of one or more liver-derived cells into one or more non-natively occurring myogenic cells suitable for consumption by contacting the population of liver-derived cells with a differentiation media,
wherein the non-natively myogenic cell suitable for consumption comprises at least 1.5-fold greater total protein (w/w%) as compared to cells not exposed to the growth media, the suspension culture, the differentiation media, or a combination thereof.

2. The method of claim 1, further comprising prior to step (a):
obtaining the population of liver-derived cells from a liver of an animal, wherein the animal is selected from a livestock, a poultry, a game, or an aquatic species.

3. The method of claim 2, wherein the liver of an animal is an embryonic liver, a fetal liver, or a neo-natal liver.

4. The method of claim 2, wherein the age of the animal is about embryonic day 16 (E16).

5. The method of any one of claims 1-4, wherein step (a) further comprises adhering the population of liver-derived cells to a substrate, wherein the population of liver-derived cells in step (a) comprises a subpopulation adhered to the substrate, a subpopulation that is non-adherent, or both.

6. The method of any one of claims 1-5, wherein the contacting of the population of liver derived cells with the growth media results in an increase in the cell proliferation rate of the population of liver-derived cells, wherein the increase in cell proliferation rate enables a population doubling level (PDL) (i.e., total number of times the cells in the population have doubled since their primary isolation *in vitro*) of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the population of liver-derived cells.

7. The method of any one of claims 1-6, wherein inducing transdifferentiation of a liver-derived cell by contacting the population of liver-derived cells with a differentiation media results in an increase in the cell proliferation rate of the population of liver-derived cells, wherein the increase in cell proliferation rate enables a PDL of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 or more passages for the population of liver-derived cells.

8. The method of any one of claims 1-7, further comprising:
an immortalizing step, wherein the population of liver-derived cells, the non-natively occurring myogenic cells, or both are immortalized,
wherein the immortalization is selected from a method comprising: transducing with a polynucleotide encoding TERT, transducing with a polynucleotide encoding CDK4/6, transducing with a polynucleotide Cyclin D1, inactivating a gene encoding an inhibitor of cyclin-dependent kinase 4/6 (CDK4/6), inactivating a gene encoding an inhibitor of Cyclin D1, or a combination thereof.

9. The method of any one of claims 1-8, further comprising:
adhering the population of liver-derived cells, non-natively occurring myogenic cells, or both to a cultivation infrastructure, wherein the adhering step comprises adhering the cells to cultivation infrastructure in the presence of differentiation medium.

10. The method of any one of claims 1-9, wherein the population of non-natively occurring myogenic cells suitable for consumption have higher proliferation rates as compared to (i) a myogenic cell not exposed to the method of claim 1 or (ii) chicken fibroblasts grown in adherent conditions.

11. The method of any one of claims 1-10, wherein the population non-natively occurring myogenic cells exhibits increased Myogenin expression, increases MyHC expression, and increased myotube formation, or a combination thereof, as compared to (i) a myogenic cell not exposed to the method of claim 1 or (ii) chicken fibroblasts grown in adherent conditions.

12. The method of any one of claims 1-11, wherein the growth media is selected from:
(i)DMEM/F12, about 20% FBS, and about 5% chicken serum;
(ii) DMEM/F12, about 20% FBS, about 5% chicken serum, CHIR99021, A-83-01, and LDN193189; and
(iii) DMEM/F12, about 10% FBS, and about 5% chicken serum.

13. The method of any one of claims 1-12, wherein the differentiation media is selected from:
(i) DMEM/F12, about 5% FBS, and about 5% Chicken serum; and
(ii) DMEM/F12, about 2% Horse serum, and about 1% ITS (Insulin-Transferrin-Selenium).

14. The method of any one of claims 1-13, wherein the population of liver-derived cells are obtained from *Gallus gallus*, *Bos taurus*, *Sous scrofa*, *Meleagris gallopavo*, *Anas platyrynchos, Salmo salar, Thunnus thynnus, Ovis aries, Coturnix coturnix, Capra aegagrus hircus*, or *Homarus americanus.*

15. The method of any one of claims 1-14, further comprising:
forming a plurality of the one or more non-natively myogenic cells into a comestible food product.
